# EUROPEAN PATENT APPLICATION

(11) **EP 4 769 462 A1**
(43) Date of publication of application: **01.07.2026**
(21) Application number: 25227150.7
(22) Date of filing: 24.12.2025
(51) Int. Cl.: H01F 38/18, A61B 6/00, H01F 27/36, H05G 1/10

(54) **X-RAY IMAGING APPARATUS, AND WIRELESS POWER TRANSFER DEVICE THEREOF**

(30) Priority: 31.12.2024 CN 202411997425; 31.12.2024 CN 202411996745
(71) Applicant: Wuhan United Imaging Healthcare Co., Ltd., WuHan, Hubei 430206 (CN)
(72) Inventor: DUAN, Yaoqiang, Wuhan, 430206 (CN); PAN, Junpeng, Wuhan, 430206 (CN); LI, Shaojie, Wuhan, 430206 (CN); ZHAO, Fanfei, Wuhan, 430206 (CN); ZHANG, Hao, Wuhan, 430206 (CN); ZHANG, Tieshan, Wuhan, 430206 (CN)
(74) Representative: Dai, Simin

(57) **Abstract**

The present disclosure provides an X-ray imaging apparatus, and a wireless power transfer device thereof. The wireless power transfer device, comprising: a first annular structure (60, 10), on which a first frame (61, 11) is fixed, a first winding (62, 12) being wound around the first frame (61, 11); and a second annular structure (63, 13), on which a second frame (64, 14) is fixed, a second winding (65, 15) being wound around the second frame (64, 14), wherein the first winding (62, 12) and the second winding (65, 15) are disposed facing each other, and the second annular structure (63, 13) is rotatable relative to the first annular structure (60, 10). The present disclosure can enhance the signal transmission performance of the X-ray imaging apparatus.

## Description

The present disclosure claims the priority of Chinese patent application No. 2024119967458 filed on December 31, 2024, and Chinese patent application No. 2024119974254 filed on December 31, 2024. The contents of the Chinese patent applications are incorporated herein by reference in their entireties.

### TECHNICAL FIELD

The present disclosure relates to the technical field of medical apparatuses, and particularly to an X-ray imaging apparatus, and a power distribution unit and a wireless power transfer device thereof.

### BACKGROUND

In recent years, X-ray computed tomography (CT) has made tremendous progress in both basic technology and new clinical applications, and has become one of the most exciting diagnostic methods in the field of medical imaging. X-ray computed tomography requires the transfer of electrical energy between a stator and a rotor. Traditional slip rings employ contact-type carbon brush slip rings, which are prone to deformation under high- and low-temperature operating conditions, face challenges in integrated molding, and tend to generate dust accumulation, carbon buildup, and heat generation during high-speed operation. Non-contact slip rings employ wireless power transfer technology to shift power transfer from power-frequency conduction to high-frequency magnetic coupling to overcome the shortcomings of traditional carbon brush slip rings, and are currently a focus of technological attention.

### SUMMARY

Some embodiments of the present disclosure provide an X-ray imaging apparatus, and a power distribution unit and a wireless power transfer device.

In a first aspect, a power distribution unit is provided, which is applied to an X-ray imaging apparatus further including a wireless power transfer device, the power distribution unit including:
a first rectifier circuit, a charging circuit, an energy storage module, and a first inverter circuit,
wherein input terminals of the first rectifier circuit and input terminals of the charging circuit are both externally connected to a power grid;
wherein an output terminal of the first rectifier circuit is connected to an input terminal of the first inverter circuit, and an output terminal of the first inverter circuit is connected to the wireless power transfer device;
wherein an output terminal of the charging circuit is connected to one end of the energy storage module, and the other end of the energy storage module is connected to the input terminal of the first inverter circuit; and
wherein the first inverter circuit is configured to convert the direct current voltage into an alternating current voltage and output the alternating current voltage to the wireless power transfer device.

Optionally, in response to a capacity of the power grid being greater than a capacity threshold, the first rectifier circuit rectifies an alternating current voltage supplied by the power grid, and outputs a direct current voltage to the first inverter circuit; and the charging circuit charges the energy storage module; and
in response to the capacity of the power grid being less than or equal to the capacity threshold, the energy storage module outputs a direct current voltage to the first inverter circuit.

Optionally, the energy storage module includes: a rechargeable battery, a bidirectional DC/DC converter, and a supercapacitor,
wherein an input terminal of the rechargeable battery is connected to the output terminal of the charging circuit; and an output terminal of the rechargeable battery is connected to one end of the supercapacitor via the bidirectional DC/DC converter, and the other end of the supercapacitor is connected to the input terminal of the first inverter circuit.

Optionally, the power distribution unit further includes: a first switch, via which the input terminal of the charging circuit is externally connected to the power grid, a control terminal of the first switch being connected to a battery management system included in the power distribution unit;
and/or the energy storage module further includes: a second switch, via which one end of the rechargeable battery is connected to the output terminal of the charging circuit, a control terminal of the second switch being connected to the battery management system included in the power distribution unit;
and/or the energy storage module further includes: a third switch, via which the other end of the rechargeable battery is connected to the bidirectional DC/DC converter, a control terminal of the third switch being connected to the battery management system included in the power distribution unit;
and/or the energy storage module further includes: a fourth switch, via which the bidirectional DC/DC converter is connected to one end of the supercapacitor, a control terminal of the fourth switch being connected to the battery management system included in the power distribution unit;
and/or the energy storage module further includes: a fifth switch, via which the other end of the supercapacitor is connected to the input terminal of the first inverter circuit, a control terminal of the fifth switch being connected to the battery management system included in the power distribution unit.

Optionally, the power distribution unit further includes a second inverter circuit;
the other end of the supercapacitor is further connected to an input terminal of the second inverter circuit; and
an output terminal of the second inverter circuit is connected to the wireless power transfer device.

Optionally, the power distribution unit further includes a second inverter circuit, a second rectifier circuit, and a first DC/AC converter;
an input terminal of the second rectifier circuit is externally connected to the power grid, an output terminal of the second rectifier circuit is connected to an input terminal of the second inverter circuit, and an output terminal of the second inverter circuit is connected to the wireless power transfer device;
the input terminal of the second rectifier circuit is further connected to the rechargeable battery via the first DC/AC converter;
in response to a capacity of the power grid being greater than a capacity threshold, the second rectifier circuit rectifies an alternating current voltage supplied by the power grid, and outputs a direct current voltage to the second inverter circuit; and
in response to the capacity of the power grid being less than or equal to the capacity threshold, the first DC/AC converter converts a direct current voltage output by the rechargeable battery into an alternating current voltage and outputs the alternating current voltage to the second rectifier circuit.

Optionally, the X-ray imaging apparatus further includes a third rectifier circuit, a third inverter circuit, and a rotating-side load; the wireless power transfer device includes a stationary-side winding and a rotating-side winding; an input terminal of the third rectifier circuit is connected to the rotating-side winding, an output terminal of the third rectifier circuit is connected to an input terminal of the third inverter circuit, and an output terminal of the third inverter circuit is connected to the rotating-side load;
the power distribution unit further includes a second DC/AC converter; and
an input terminal of the second DC/AC converter is connected to the output terminal of the energy storage module and/or to the output terminal of the first rectifier circuit, and an output terminal of the second DC/AC converter is connected to the stationary-side winding.

Optionally, when the energy storage module includes a rechargeable battery, the power distribution unit further includes a third DC/AC converter, via which the rechargeable battery is connected to the input terminal of the first rectifier circuit.

In a second aspect, provided is a wireless power transfer device, including:
a first annular structure, on which a first frame is fixed, a first winding being wound around the first frame; and
a second annular structure, on which a second frame is fixed, a second winding being wound around the second frame,
wherein the first winding and the second winding are disposed facing each other, and the second annular structure is rotatable relative to the first annular structure.

Optionally, the first frame is fixed to an axial side surface of the first annular structure, and the second frame is fixed to an axial side surface of the second annular structure; and
a gap exists between the first winding and the second winding.

Optionally, the wireless power transfer device includes at least two first windings, part of the at least two first windings being main windings and the remaining first windings being auxiliary windings; the wireless power transfer device includes at least two second windings, part of the at least two second windings being main windings and the remaining second windings being auxiliary windings;
and/or a magnetic core assembly is further mounted to an axial side surface of the first annular structure, the magnetic core assembly including at least one magnetic core, the first winding passing through a window of the magnetic core, and the second frame passing through an air gap of the magnetic core and being fixed to the second annular structure;
and/or the first annular structure and the second annular structure have the same dimensions.

Optionally, the wireless power transfer device further includes:
a metal shielding assembly disposed relative to the first winding and/or the second winding.

Optionally, the first frame, around which the first winding is wound, is fixed to a first axial side surface of the first annular structure;
the metal shielding assembly includes a first metal shielding plate disposed on a second axial side surface of the first annular structure;
and/or the metal shielding assembly includes a second metal shielding plate; the second frame, around which the second winding is wound, is fixed to a first axial side surface of the second annular structure, and the second metal shielding plate is disposed on a second axial side surface of the second annular structure;
and/or the metal shielding assembly includes a first metal shielding ring structure; and the wireless power transfer device includes a plurality of first windings, which are circumferentially arranged on the first axial side surface of the first annular structure to form a power ring, the first metal shielding ring structure being fitted over at least one circumferential surface of the power ring, wherein an axis of the power ring coincides with an axis of the first annular structure.

Optionally, when the metal shielding assembly includes the first metal shielding ring structure and the first metal shielding plate, an axial side surface of the first metal shielding ring structure close to the first metal shielding plate is seamlessly connected to the first metal shielding plate; and
when the metal shielding assembly includes the first metal shielding ring structure and the second metal shielding plate, an axial side surface of the first metal shielding ring structure close to the second metal shielding plate is seamlessly connected to the second metal shielding plate.

Optionally, a magnetic core assembly is further mounted to the first axial side surface of the first annular structure, the magnetic core assembly including at least one magnetic core, the first winding passing through a window of the magnetic core, and the second frame passing through an air gap of the magnetic core and being fixed to the second annular structure;
the area of an axial projection of the first metal shielding plate is greater than or equal to the area of a projection of the magnetic core on an axial side surface of the first metal shielding plate;
and/or the area of an axial projection of the second metal shielding plate is greater than or equal to the area of a projection of the magnetic core on an axial side surface of the second metal shielding plate;
and/or the area of a radial projection of the first metal shielding ring structure is greater than or equal to the area of a projection of the magnetic core on a circumferential surface of the first metal shielding ring structure.

Optionally, the first frame, around which the first winding is wound, is located on a first circumferential surface of the first annular structure; the metal shielding assembly includes a second metal shielding ring structure; the second metal shielding ring structure is fitted over a second circumferential surface of the first annular structure and/or a first circumferential surface of the second annular structure; the second frame is fixed to a second circumferential surface of the second annular structure;
and/or each circumferential surface of the first annular structure is provided with a first frame, and one second annular structure is provided opposite each circumferential surface of the first annular structure, a second frame being fixed to the first circumferential surface of each second annular structure relative to the first frame; the metal shielding assembly includes a third metal shielding ring structure; one third metal shielding ring structure is fitted over the second circumferential surface of at least one of the second annular structures;
and/or the metal shielding assembly includes a third metal shielding plate; and the third metal shielding plate is disposed opposite a first and/or second axial side surface of the first annular structure.

Optionally, when the metal shielding assembly includes the second metal shielding ring structure and the third metal shielding plate, an axial side surface of the second metal shielding ring structure is seamlessly connected to the third metal shielding plate; and
when the metal shielding assembly includes the third metal shielding ring structure and the third metal shielding plate, an axial side surface of the third metal shielding ring structure is seamlessly connected to the third metal shielding plate.

Optionally, a magnetic core assembly is further mounted to the first axial side surface of the first annular structure, the magnetic core assembly including at least one magnetic core, the first winding passing through a window of the magnetic core, and the second frame passing through an air gap of the magnetic core and being fixed to the second annular structure;
the area of an axial projection of the third metal shielding plate is greater than or equal to the area of a projection of the magnetic core on an axial side surface of the third metal shielding plate;
and/or the area of a radial projection of the second metal shielding ring structure is greater than or equal to the area of a projection of the magnetic core on a circumferential surface of the second metal shielding ring structure;
and/or the area of a radial projection of the third metal shielding ring structure is greater than or equal to the area of a projection of the magnetic core on a circumferential surface of the third metal shielding ring structure.

Optionally, the metal shielding assembly is made of aluminum;
and/or a magnetic core assembly is further mounted to a first axial side surface of the first annular structure, the magnetic core assembly including at least one magnetic core, the first winding passing through a window of the magnetic core, and the second frame passing through an air gap of the magnetic core and being fixed to the second annular structure; and the thickness of the metal shielding assembly is negatively correlated with the number of magnetic cores;
and/or the first annular structure is configured for fixed connection with a gantry, and is located on a stationary side of the gantry.

In a third aspect, provided is an X-ray imaging apparatus, including a power distribution unit and/or a wireless power transfer device,
wherein the power distribution unit includes:
a first rectifier circuit, a charging circuit, an energy storage module, and a first inverter circuit,
wherein input terminals of the first rectifier circuit and input terminals of the charging circuit are both externally connected to a power grid;
wherein an output terminal of the first rectifier circuit is connected to an input terminal of the first inverter circuit, and an output terminal of the first inverter circuit is connected to the wireless power transfer device;
wherein an output terminal of the charging circuit is connected to one end of the energy storage module, and the other end of the energy storage module is connected to the input terminal of the first inverter circuit;
wherein the first inverter circuit is configured to convert an output voltage of the first rectifier circuit into an alternating current voltage and output the alternating current voltage to the wireless power transfer device; and
wherein the wireless power transfer device includes:
   a first annular structure, on which a first frame is fixed, a first winding being wound around the first frame; and
   a second annular structure, on which a second frame is fixed, a second winding being wound around the second frame,
   wherein the first winding and the second winding are disposed facing each other, and the second annular structure is rotatable relative to the first annular structure.

Optionally, the X-ray imaging apparatus includes the wireless power transfer device of any item of the second aspect, and a gantry, wherein the first annular structure is fixedly connected to the gantry and is located on a stationary side of the gantry, and the second annular structure is located on a rotating side of the gantry.

Optionally, the power distribution unit is connected to the first winding.

Based on common knowledge in the art, the above preferred conditions can be combined arbitrarily to obtain various instances of the present disclosure or their variations or modifications.

The positive and progressive effects of some embodiments of the present disclosure are as follows: In the present disclosure, the power grid and the energy storage module supply power in parallel, so that the demand of the X-ray imaging apparatus on the capacity of the power grid can be reduced. Since the charging circuit only participates in the charging process of the energy storage module and not in the discharging process of the energy storage module, the energy storage module can be "charged and discharged simultaneously". Moreover, there are no excessive requirements on the power rating of the charging circuit, so that a charging circuit on the order of several kilowatts is sufficient. This can reduce the cost of the charging circuit and thus reduce the overall cost of the power distribution unit.

In some embodiments of the present disclosure, a metal shielding assembly is added to the wireless power transfer device, and can utilize the eddy current effect of the metal material to weaken the main magnetic flux of the wireless power transfer device and reduce the leakage of the magnetic flux lines, thereby reducing electromagnetic interference. This mitigates the impact of electromagnetic interference on the communication interaction of the X-ray imaging apparatus, reduces image distortion during scanning of the X-ray imaging apparatus, and also mitigates potential harm to the human body caused by excessive magnetic flux density at the rotation center of the wireless power transfer device and alleviates the heat generation problem caused by the eddy current effect.

### BRIEF DESCRIPTION OF THE DRAWINGS

FIG. 1 is a structural schematic diagram of an X-ray imaging apparatus according to the prior art, wherein M1 represents a power grid, M2 represents an inverter circuit, M3 represents a bidirectional DC/DC converter, M4 represents an energy storage unit, M5 represents a filament control unit, M6 represents an anode driver.
FIG. 2 is a structural schematic diagram of the configuration of a power distribution unit according to an exemplary embodiment of the present disclosure.
FIG. 3 is a schematic diagram of an application scenario of a power distribution unit according to an exemplary embodiment of the present disclosure, wherein M1 represents a power grid, M5 represents a filament control unit, M6 represents an anode driver.
FIG. 4 is a schematic diagram of an application scenario of another power distribution unit according to an exemplary embodiment of the present disclosure, wherein M1 represents a power grid, M5 represents a filament control unit, M6 represents an anode driver.
FIG. 5 is a schematic diagram of an application scenario of another power distribution unit according to an exemplary embodiment of the present disclosure, wherein M1 represents a power grid, M5 represents a filament control unit, M6 represents an anode driver, M7 represents a rotating-side step-up transformer, M8 represents a rotating-side rectifier-filter module, M11 represents a rechargeable battery, M10 represents a bidirectional DC/DC converter, M9 represents a supercapacitor.
FIG. 6 is a cross-sectional view of a wireless power transfer device of an X-ray imaging apparatus according to an exemplary embodiment of the present disclosure.
FIG. 7 is a cross-sectional view of a wireless power transfer device of another X-ray imaging apparatus according to an exemplary embodiment of the present disclosure.
FIG. 8 is a structural schematic diagram of a wireless power transfer device with an added metal shielding assembly according to an exemplary embodiment of the present disclosure.
FIG. 9 is a structural schematic diagram of another wireless power transfer device with an added metal shielding assembly according to an exemplary embodiment of the present disclosure.
FIG. 10 is a structural schematic diagram of another wireless power transfer device with an added metal shielding assembly according to an exemplary embodiment of the present disclosure.
FIG. 11 is a structural schematic diagram of another wireless power transfer device with an added metal shielding assembly according to an exemplary embodiment of the present disclosure.
FIG. 12 is a structural schematic diagram of another wireless power transfer device with an added metal shielding assembly according to an exemplary embodiment of the present disclosure.
FIG. 13 is a structural schematic diagram of another wireless power transfer device with an added metal shielding assembly according to an exemplary embodiment of the present disclosure.
FIG. 14 is a structural schematic diagram of another wireless power transfer device with an added metal shielding assembly according to an exemplary embodiment of the present disclosure.
FIG. 15 is a structural schematic diagram of another wireless power transfer device with an added metal shielding assembly according to an exemplary embodiment of the present disclosure.
FIG. 16 is a structural schematic diagram of another wireless power transfer device with an added metal shielding assembly according to an exemplary embodiment of the present disclosure.
FIG. 17 is a structural schematic diagram of another wireless power transfer device with an added metal shielding assembly according to an exemplary embodiment of the present disclosure.
FIG. 18 is a structural schematic diagram of another wireless power transfer device with an added metal shielding assembly according to an exemplary embodiment of the present disclosure.
FIG. 19 is a structural schematic diagram of another wireless power transfer device with an added metal shielding assembly according to an exemplary embodiment of the present disclosure.
FIG. 20 is a structural schematic diagram of another wireless power transfer device with an added metal shielding assembly according to an exemplary embodiment of the present disclosure.
FIG. 21 is a structural schematic diagram of another wireless power transfer device with an added metal shielding assembly according to an exemplary embodiment of the present disclosure.
FIG. 22 is a structural schematic diagram of another wireless power transfer device with an added metal shielding assembly according to an exemplary embodiment of the present disclosure.
FIG. 23 is a structural schematic diagram of another wireless power transfer device with an added metal shielding assembly according to an exemplary embodiment of the present disclosure.
FIG. 24 is a structural schematic diagram of another wireless power transfer device with an added metal shielding assembly according to an exemplary embodiment of the present disclosure.
FIG. 25 is a schematic diagram of the effect of magnetic flux lines generated by another wireless power transfer device without an added metal shielding assembly according to an exemplary embodiment of the present disclosure.
FIG. 26 is an axial schematic diagram of a wireless power transfer device without an added metal shielding assembly according to an exemplary embodiment of the present disclosure.
FIG. 27 is a schematic diagram of the effect of magnetic flux lines generated by a wireless power transfer device without an added metal shielding assembly according to an exemplary embodiment of the present disclosure.
FIG. 28 is a schematic connection diagram of a first switch of a power distribution unit according to an exemplary embodiment of the present disclosure.
FIG. 29 is a schematic connection diagram of a second switch of a power distribution unit according to an exemplary embodiment of the present disclosure.
FIG. 30 is a schematic connection diagram of a third switch of a power distribution unit according to an exemplary embodiment of the present disclosure.
FIG. 31 is a schematic connection diagram of a fourth switch of a power distribution unit according to an exemplary embodiment of the present disclosure.
FIG. 32 is a schematic connection diagram of a fifth switch of a power distribution unit according to an exemplary embodiment of the present disclosure.
FIG. 33 is a schematic connection diagram of a sixth switch of a power distribution unit according to an exemplary embodiment of the present disclosure.
FIG. 34 is a schematic connection diagram of a seventh switch of a power distribution unit according to an exemplary embodiment of the present disclosure.
FIG. 35 is a schematic connection diagram of an eighth switch of a power distribution unit according to an exemplary embodiment of the present disclosure.
FIG. 36 is a schematic connection diagram of a ninth switch of a power distribution unit according to an exemplary embodiment of the present disclosure.
FIG. 37 is a schematic connection diagram of a tenth switch of a power distribution unit according to an exemplary embodiment of the present disclosure.
FIG. 38 is a schematic diagram of the overall structure of a non-contact slip ring structure in the prior art.
FIG. 39 is a side view of the non-contact slip ring structure in the prior art.
FIG. 40 is a cross-sectional view of the non-contact slip ring structure in the prior art.

### DETAILED DESCRIPTION

The present disclosure is further illustrated below by way of embodiments, but is not thus limited within the scope of the embodiments.

In the embodiments of the present disclosure, prefixes such as "first" and "second" are used merely to distinguish different descriptive objects and do not limit the position, order, priority, quantity, or content of the described objects. The use of prefixes such as ordinal numbers to distinguish descriptive objects in the embodiments of the present disclosure does not constitute a limitation on the described objects. The description of the described objects shall refer to the contextual description in the claims or embodiments, and the use of such prefixes should not constitute an unnecessary limitation. In addition, in the description of the embodiments, "a plurality of" means two or more, unless otherwise stated.

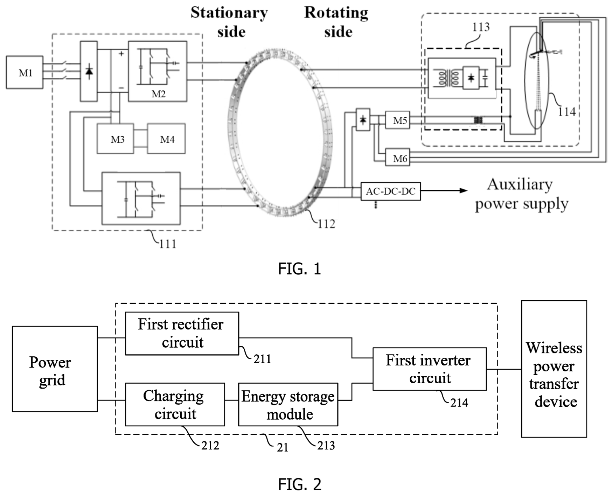

FIG. 1 is a structural schematic diagram of an X-ray imaging apparatus according to the prior art, wherein M1 represents a power grid, M2 represents an inverter circuit, M3 represents a bidirectional DC/DC converter, M4 represents an energy storage unit, M5 represents a filament control unit, M6 represents an anode driver. The X-ray imaging apparatus mainly includes a power distribution unit (PDU) 111, a wireless power transfer device 112, a high voltage generator (HVG) 113, an X-ray tube 114, a detector, and a gantry (not shown). The power distribution unit 111 is located on a stationary side of the gantry, and the HVG 113, the X-ray tube 114 and the detector are located on a rotating side of the gantry. The electrical energy output by the power distribution unit 111 powers the HVG 113, the detector, and other components via the wireless power transfer device 112. The HVG 113 converts low-voltage electricity into high-voltage direct current (up to 140kV) and applies the high-voltage direct current between an anode and a cathode of the X-ray tube 114, to generate a high-voltage electric field between the anode and the cathode of the X-ray tube 114. Simultaneously, the HVG 113 provides current to a filament of the cathode of the X-ray tube 114, causing the filament to heat up and generate free electrons at the cathode. Under the action of the high-voltage electric field, the electrons bombard an anode target at high speed, generating X-rays. In additional, the HVG 113 supplies anode drive power to an anode motor of the X-ray tube 114, enabling the anode target to rotate at high speed and facilitating heat dissipation from the anode target. The detector converts the X-rays generated by the X-ray tube 114 into image signals, enabling scan imaging.

In some embodiments of the present disclosure, for a CT employing a wireless power transfer device, the wireless power transfer device may be powered by the power distribution unit. In some embodiments of the present disclosure, to address the problem of insufficient capacity of the power grid at the installation site, an energy storage unit may be added to the power distribution unit 111.

For a CT employing a wireless power transfer device, the power distribution unit 111 includes a rectifier circuit, an inverter circuit, a bidirectional DC/DC converter, and an energy storage unit. The energy storage unit is connected to an output terminal of the rectifier circuit and an input terminal of the inverter circuit via the bidirectional DC/DC converter. The charging and discharging processes of the energy storage unit are both performed via the bidirectional DC/DC converter. As a result, simultaneous charging and discharging is impossible, and when the energy storage unit discharges to power components included in the power distribution unit, the power of the bidirectional DC/DC converter should not be less than the sum of the power of all the power components. Taking a power component including a first inverter circuit and a second inverter circuit as an example, the power of the bidirectional DC/DC converter should not be less than the sum of the power of the first inverter circuit (on the order of hundreds of kilowatts) and the second inverter circuit (on the order of tens of kilowatts). This places high demands on the power rating of the bidirectional DC/DC converter, thereby significantly increasing the design difficulty and cost of the bidirectional DC/DC converter.

To address the aforementioned shortcomings, the embodiments of the present disclosure provide a low-cost power distribution unit capable of enabling "simultaneous charging and discharging" of an energy storage module.

FIG. 2 is a structural schematic diagram of the configuration of a power distribution unit according to an exemplary embodiment of the present disclosure. The power distribution unit 21 includes: a first rectifier circuit 211, a charging circuit 212, an energy storage module 213, and a first inverter circuit 214.

Input terminals of the first rectifier circuit 211 and the charging circuit 212 are both externally connected to a power grid. An output terminal of the first rectifier circuit 211 is connected to an input terminal of the first inverter circuit 214, and an output terminal of the first inverter circuit 214 is connected to a wireless power transfer device. An output terminal of the charging circuit 212 is connected to one end of the energy storage module 213 and the other end of the energy storage module 213 is connected to the input terminal of the first inverter circuit 214. Specifically, the wireless power transfer device includes a stationary-side winding and a rotating-side winding, and the output terminal of the first inverter circuit 214 is connected to the stationary-side winding. In this embodiment, the main power inversion is implemented on the stationary side.

In this embodiment, the power grid and the energy storage module supply power in parallel, so that the demand of the X-ray imaging apparatus on the capacity of the power grid can be reduced. The charging circuit 212 uses the power grid to charge the energy storage module 213, and the energy storage module 213 can power the first inverter circuit 214. Therefore, the charging circuit and a discharging circuit of the energy storage module 213 are separated. Since the charging circuit only participates in the charging process of the energy storage module and not in the discharging process of the energy storage module, the energy storage module can be "charged and discharged simultaneously". Moreover, there are no excessive requirements on the power rating of the charging circuit, so that a charging circuit on the order of several kilowatts is sufficient. This can reduce the cost of the charging circuit and thus reduce the overall cost of the power distribution unit.

In one embodiment, in response to the capacity of the power grid being greater than a capacity threshold, the first rectifier circuit 211 rectifies an alternating current voltage supplied by the power grid, and outputs a direct current voltage to the first inverter circuit 214. The first inverter circuit 214 is used to convert the direct current voltage to an alternating current voltage and output the alternating current voltage to the wireless power transfer device, to power the wireless power transfer device and therefore other loads (e.g., an HVG, and a detector) of the X-ray imaging apparatus.

In one embodiment, in response to the capacity of the power grid being greater than the capacity threshold, the charging circuit 212 charges the energy storage module 213 using the alternating current voltage supplied by the power grid. Further, the energy storage module 213 can power the first inverter circuit 214, thus enabling both the power grid and the energy storage module 213 to simultaneously power the wireless power transfer device via the first inverter circuit 214, ensuring power supply stability.

In response to the capacity of the power grid being less than or equal to the capacity threshold, the energy storage module 213 outputs a direct current voltage to the first inverter circuit 214, and the first inverter circuit 214 powers the wireless power transfer device. In this case, the energy storage module 213 powers the wireless power transfer device solely, or the energy storage module 213 primarily powers the wireless power transfer device. When the energy storage module 213 outputs the direct current voltage to the first inverter circuit 214, the direct current voltage is not required to pass through the charging circuit 212. That is, the charging circuit 212 does not participate in the discharging process of the energy storage module 213, thus enabling the energy storage module 213 to be "charged and discharged simultaneously".

The capacity threshold can be set according to actual requirements. The capacity of the power grid being greater than the capacity threshold indicates that the capacity of the power grid is sufficient to power the X-ray imaging apparatus. The capacity of the power grid being less than or equal to the capacity threshold indicates that the capacity of the power grid is insufficient to power the X-ray imaging apparatus, and it is necessary to use the power grid and the energy storage module to jointly power the X-ray imaging apparatus. Alternatively, it is necessary to switch to an off-grid mode, wherein the energy storage module powers the X-ray imaging apparatus solely, so that the energy storage module can ensure the safe and stable operation of the X-ray imaging apparatus even in the event of a sudden power failure at the installation site of the X-ray imaging apparatus.

In one embodiment, the first rectifier circuit 211 and the energy storage module 213 are controlled differently according to different scanning protocols and capacity conditions of the power grid, to provide the wireless power transfer device with an alternating current voltage corresponding to a scanning protocol. Subsequently, through the transfer of the stationary-side winding and the rotating-side winding of the wireless power transfer device, and the voltage conversion of a rotating-side step-up transformer and a rotating-side rectifier-filter module, a voltage corresponding to the scanning protocol can be generated between the anode and the cathode of the X-ray tube, thereby enabling scan imaging of a corresponding part of a patient.

In one embodiment, the charging circuit 212 employs an AFE (Analog Front End) charger. An input terminal of the AFE charger is connected to a neutral (N) line of the power grid and any one of phase lines A, B, or C. The AFE charger enables efficient and reliable charging of the energy storage module 213.

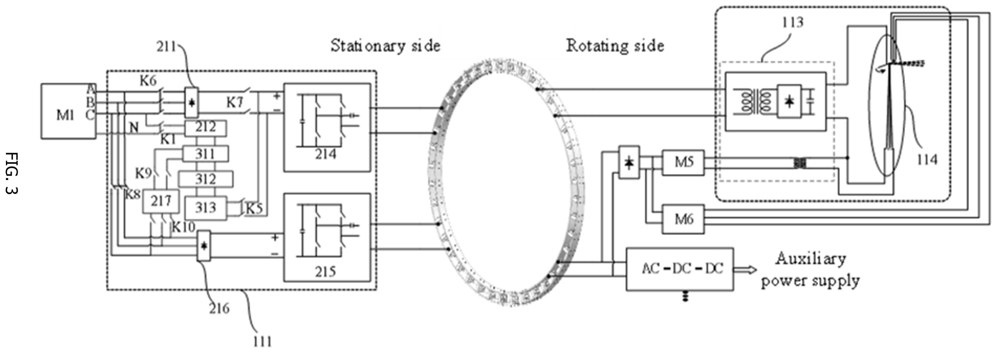

In one embodiment, referring to FIG. 3, wherein M1 represents a power grid, M5 represents a filament control unit, M6 represents an anode driver, the energy storage module 213 includes: a rechargeable battery 311, a bidirectional DC/DC converter 312, and a supercapacitor 313. The rechargeable battery 311 may be, but is not limited to, a lithium battery.

An input terminal of the rechargeable battery 311 is connected to the output terminal of the charging circuit 212. An output terminal of the rechargeable battery 311 is connected to one end of the supercapacitor 313 via the bidirectional DC/DC converter 312, and the other end of the supercapacitor 313 is connected to the input terminal of the first inverter circuit 214.

The charging circuit 212 first charges the rechargeable battery 311, the rechargeable battery 311 charges the supercapacitor 313 via the bidirectional DC/DC converter 312, and the supercapacitor 313 then powers the first inverter circuit 214.

The bidirectional DC/DC converter 312 is a device that enables electrical energy conversion, allowing a direct current voltage to flow bidirectionally, that is, the direct current voltage can flow from the input side to the output side, and vice versa. Therefore, the rechargeable battery 311 can also recover energy from braking of a rotating-side motor or heat loss of the X-ray tube via the bidirectional DC/DC converter 312, thereby improving energy utilization efficiency and meeting the requirements for energy conservation and emission reduction. Specifically, for energy recovery from the braking of the rotating-side motor, regenerative braking technology may be considered. During braking, a motor controller changes the operating mode of the rotating-side motor to a generator mode, such that alternating current electrical energy generated by the motor is reversibly converted into direct current electrical energy via a motor drive circuit and stored in a rotating-side direct current capacitor. In this case, by replacing an uncontrolled rectifier module on the rotating side with an active rectifier module, the electrical energy output from the rotating-side motor can be transferred to the stationary-side supercapacitor for storage via a wireless slip ring, and this stored energy is then transferred to the rechargeable battery 311 for energy recovery via the bidirectional DC/DC converter 312. For energy recovery from the heat loss of the rotating-side X-ray tube, two approaches may be considered. In one approach, the heat energy dissipated from the X-ray tube is recovered, the heat energy is then converted into direct current power via a thermoelectric module, the direct current electrical energy on the rotating side is then transferred to the stationary-side supercapacitor for storage via the active rectifier module and the wireless slip ring on the rotating side, and this stored energy is then transferred to the rechargeable battery 311 for energy recovery via the bidirectional DC/DC converter 312. Alternatively, the heat energy is converted into mechanical energy via a heat engine, the mechanical energy is then converted into alternating current power via a generator, the alternating current power is rectified into direct current, the direct current electrical energy on the rotating side is then transferred to the stationary-side supercapacitor for storage via the active rectifier module and the wireless slip ring on the rotating side, and finally this stored energy is transferred to the rechargeable battery 311 for energy recovery via the bidirectional DC/DC converter 312.

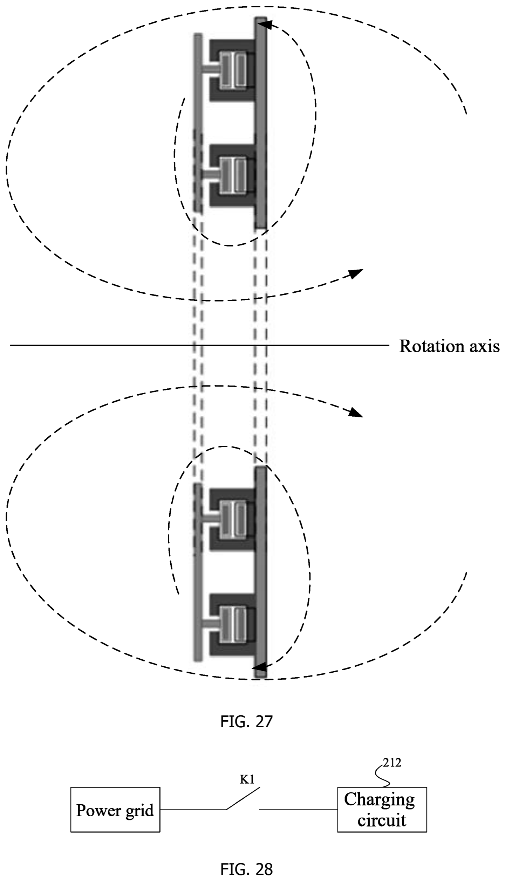

In one embodiment, referring to FIGS. 3 and 28, the power distribution unit further includes: a first switch K1. The input terminal of the charging circuit 212 is externally connected to the power grid via the first switch K1. A control terminal of the first switch K1 is connected to a battery management system included in the power distribution unit 21.

The battery management system controls the opening and closing of the first switch K1 to switch the connection state between the charging circuit 212 and the power grid. For example, in response to the remaining capacity of the rechargeable battery 311 being less than a first remaining capacity threshold, i.e., the remaining capacity of the rechargeable battery 311 is insufficient, the battery management system controls the first switch K1 to close, at which point the charging circuit 212 is connected to the power grid, and the charging circuit 212 can use the electrical energy from the power grid to charge the rechargeable battery 311. In response to the remaining capacity of the rechargeable battery 311 being greater than or equal to the first remaining capacity threshold, i.e., the remaining capacity of the rechargeable battery 311 is sufficient, the battery management system controls the first switch K1 to open, at which point the charging circuit 212 is disconnected from the power grid, and the charging circuit 212 stops charging the rechargeable battery 311. The first remaining capacity threshold is set according to actual requirements.

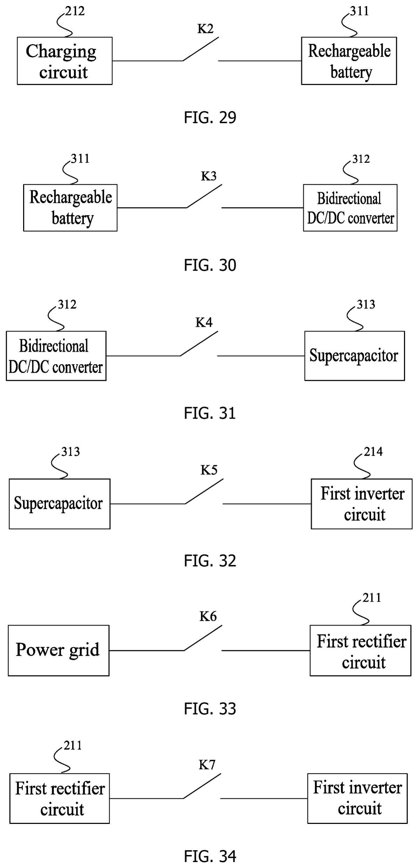

In one embodiment, referring to FIG. 29, the energy storage module 213 further includes: a second switch K2. One end of the rechargeable battery 311 is connected to the output terminal of the charging circuit 212 via the second switch K2. A control terminal of second switch K2 is connected to the battery management system included in the power distribution unit 21.

The battery management system controls the opening and closing of the second switch K2 to switch the connection state between the charging circuit 212 and the rechargeable battery 311. For example, in response to the remaining capacity of the rechargeable battery 311 being less than the first remaining capacity threshold, i.e., the remaining capacity of the rechargeable battery 311 is insufficient, the battery management system controls the second switch K2 to close, at which point the charging circuit 212 is connected to the rechargeable battery 311, and the charging circuit 212 can charge the rechargeable battery 311. In response to the remaining capacity of the rechargeable battery 311 being greater than or equal to the first remaining capacity threshold, i.e., the remaining capacity of the rechargeable battery 311 is sufficient, the battery management system controls the second switch K2 to open, at which point the charging circuit 212 is disconnected from the rechargeable battery 311, and the charging circuit 212 stops charging the rechargeable battery 311.

In one embodiment, referring to FIG. 30, the energy storage module 213 further includes: a third switch K3. The other end of the rechargeable battery 311 is connected to the bidirectional DC/DC converter 312 via the third switch K3. A control terminal of the third switch K3 is connected to the battery management system included in the power distribution unit 21.

The battery management system controls the opening and closing of the third switch K3 to switch the connection state between the bidirectional DC/DC converter 312 and the rechargeable battery 311. For example, in response to the remaining capacity of the supercapacitor 313 being less than a second remaining capacity threshold, the battery management system controls the third switch K3 to close, at which point the bidirectional DC/DC converter 312 is connected to the rechargeable battery 311, and the bidirectional DC/DC converter can use the electrical energy of the rechargeable battery 311 to charge the supercapacitor 313. In response to the remaining capacity of the supercapacitor 313 being greater than or equal to the second remaining capacity threshold, the battery management system controls the third switch K3 to open, at which point the bidirectional DC/DC converter 312 is disconnected from the rechargeable battery 311, and the bidirectional DC/DC converter stops charging the rechargeable battery 311.

In one embodiment, referring to FIG. 31, the energy storage module 213 further includes: a fourth switch K4. The bidirectional DC/DC converter 312 is connected to one end of the supercapacitor 313 via the fourth switch K4. A control terminal of the fourth switch K4 is connected to the battery management system included in the power distribution unit 21.

The battery management system controls the opening and closing of the fourth switch K4 to switch the connection state between the bidirectional DC/DC converter 312 and the supercapacitor 313. For example, in response to the remaining capacity of the supercapacitor 313 being less than the second remaining capacity threshold, the battery management system controls the fourth switch K4 to close, at which point the bidirectional DC/DC converter 312 is connected to the supercapacitor 313, and the bidirectional DC/DC converter can charge the supercapacitor 313. In response to the remaining capacity of the supercapacitor 313 being greater than or equal to the second remaining capacity threshold, the battery management system controls the fourth switch K4 to open, at which point the bidirectional DC/DC converter 312 is disconnected from the supercapacitor 313, and the bidirectional DC/DC converter stops charging the supercapacitor 313. The second remaining capacity threshold can be set according to actual requirements.

In one embodiment, referring to FIG. 32, the energy storage module 213 further includes: a fifth switch K5. The other end of the supercapacitor 313 is connected to the input terminal of the first inverter circuit 214 via the fifth switch K5. A control terminal of the fifth switch K5 is connected to the battery management system included in the power distribution unit 21.

The battery management system controls the opening and closing of the fifth switch K5 to switch the connection state between the first inverter circuit 214 and the supercapacitor 313. For example, in response to the capacity of the power grid being less than or equal to the capacity threshold, the battery management system controls the fifth switch K5 to close, at which point the first inverter circuit 214 is connected to the supercapacitor 313, and the supercapacitor 313 can power the first inverter circuit 214. In response to the capacity of the power grid being greater than the capacity threshold, the battery management system controls the fifth switch K5 to open, at which point the first inverter circuit 214 is disconnected from the supercapacitor 313, and the supercapacitor 313 stops powering the first inverter circuit 214.

In one embodiment, referring to FIGS. 33 and 34, the power distribution unit further includes a sixth switch K6 and a seventh switch K7. The input terminal of the first rectifier circuit 211 is externally connected to the power grid via the sixth switch K6, and the output terminal of the first rectifier circuit 211 is connected to the input terminal of the first inverter circuit via the seventh switch K7. Control terminals of the sixth switch K6 and the seventh switch K7 are both connected to the battery management system. In response to the capacity of the power grid being greater than the capacity threshold, the battery management system controls the sixth switch K6 and the seventh switch K7 to close. The first rectifier circuit 211 rectifies an alternating current voltage supplied by the power grid, and outputs a direct current voltage to the first inverter circuit 214, such that the first inverter circuit 214 converts the direct current voltage to an alternating current voltage and outputs the alternating current voltage to the wireless power transfer device, to power the wireless power transfer device.

In one embodiment, referring to FIG. 3, the power distribution unit 21 further includes a second inverter circuit 215 and a second rectifier circuit 216. An input terminal of the second rectifier circuit 216 is externally connected to the power grid, an output terminal of the second rectifier circuit 216 is connected to an input terminal of the second inverter circuit 215, and an output terminal of the second inverter circuit 215 is connected to the wireless power transfer device.

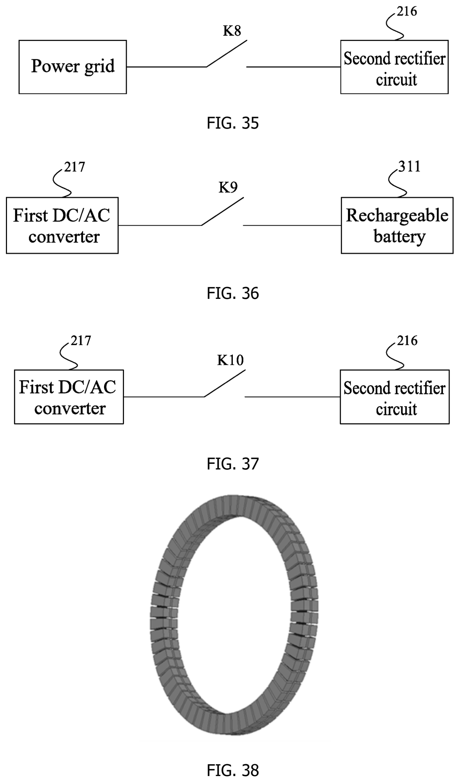

In one embodiment, referring to FIG. 35, the input terminal of the second rectifier circuit 216 is externally connected to the power grid via an eighth switch K8, and a control terminal of the eighth switch K8 is connected to the battery management system. When the operation of an auxiliary inverter circuit is required, the battery management system controls the eighth switch K8 to close, thereby connecting the second rectifier circuit 216 to the power grid.

In this embodiment, one of the two inverter circuits serves as a main inverter circuit, and the other as an auxiliary inverter circuit. The auxiliary inverter circuit can power loads (e.g., an anode drive of the X-ray tube 114) other than the main loads such as the HVG. The main inverter circuit will only start powering the main loads such as the HVG after the auxiliary inverter circuit has powered the other loads. The power of the auxiliary inverter circuit is much lower than the power rating of the main inverter circuit.

Those skilled in the art should understand that, for the circuit structure of the power distribution unit in FIG. 2 of the present disclosure, the second inverter circuit 215, the second rectifier circuit 216, and the corresponding circuit connection relationship can also be added as shown in FIG. 3. In this way, in the circuit structure of the power distribution unit in FIG. 2, one of the two inverter circuits can also serve as the main inverter circuit, and the other as the auxiliary inverter circuit.

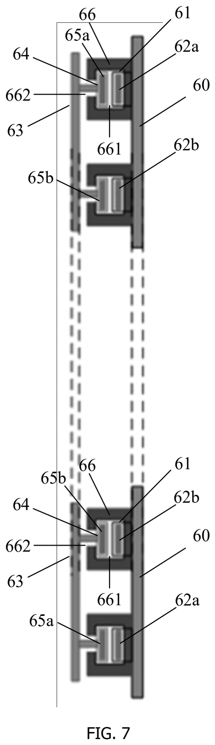

In one embodiment, referring to FIG. 7, the wireless power transfer device includes two sets of stationary-side windings. One set of stationary-side windings include a plurality of first stationary-side windings, and the other set of stationary-side windings includes a plurality of second stationary-side windings. The windings in the two sets of stationary-side windings may be arranged either regularly or irregularly. Specific arrangements are described in the following embodiments. The two inverter circuits are respectively connected to the first stationary-side windings and the second stationary-side windings, and respectively power the first stationary-side windings and the second stationary-side windings. One of the two sets of stationary-side windings may serve as main windings, and the other as auxiliary windings. Corresponding to the stationary-side windings, the wireless power transfer device includes two sets of rotating-side windings. One set of rotating-side windings includes a plurality of first rotating-side windings, and the other set of rotating-side windings includes a plurality of second rotating-side windings. One of the two sets of rotating-side windings serves as the main windings, and the other as the auxiliary windings.

In specific applications, the first inverter circuit 214 and the second inverter circuit 215 can be controlled differently according to different scanning protocols, to convert a direct current voltage into an alternating current voltage corresponding to a scanning protocol. Subsequently, through the transfer of the stationary-side winding and the rotating-side winding of the wireless power transfer device, and the voltage conversion of a rotating-side step-up transformer and a rotating-side rectifier-filter module, a voltage corresponding to the scanning protocol can be generated between the anode and the cathode of the X-ray tube, thereby enabling scan imaging of a corresponding part of the patient.

It should be noted that the number of sets of stationary-side windings is not limited to two, and the number of inverter circuits is not limited to two as shown in FIG. 3, as long as the number of inverter circuits matches the number of sets of stationary-side windings.

In one embodiment, referring to FIG. 3, the power distribution unit 21 further includes a first DC/AC converter 217. The input terminal of the second rectifier circuit 216 is also connected to the rechargeable battery 311 via the first DC/AC converter 217. In response to the capacity of the power grid being greater than the capacity threshold, the second rectifier circuit 216 rectifies an alternating current voltage supplied by the power grid, and outputs a direct current voltage to the second inverter circuit 215. In response to the capacity of the power grid being less than or equal to the capacity threshold, the first DC/AC converter 217 converts a direct current voltage output by the rechargeable battery 311 into an alternating current voltage and outputs the alternating current voltage to the second rectifier circuit 216. In this embodiment, both inverter circuits are located on the stationary side, and the second inverter circuit 215 is powered by the power grid and/or the rechargeable battery 311.

In one embodiment, referring to FIGS. 36 and 37, the power distribution unit 21 further includes a ninth switch K9 and a tenth switch K10. The first DC/AC converter 217 is connected to the rechargeable battery and the second rectifier circuit 216 via the ninth switch K9 and the tenth switch K10, respectively. Control terminals of the ninth switch K9 and the tenth switch K10 are both connected to the battery management system. In response to the capacity of the power grid being less than or equal to the capacity threshold, the battery management system controls the ninth switch K9 and the tenth switch K10 to close, and the first DC/AC converter 217 converts a direct current voltage output by the rechargeable battery 311 into an alternating current voltage and outputs the alternating current voltage to the second inverter circuit 215.

In this embodiment, similar to the first inverter circuit, both the power grid and the energy storage module can power the second inverter circuit. In specific applications, the second rectifier circuit and the first DC/AC converter can be controlled differently according to different scanning protocols and capacity conditions of the power grid to provide the required electrical energy to the second inverter circuit.

Those skilled in the art should understand that the power distribution unit 21 of the present disclosure, in its specific implementation, may include any one of the first to tenth switches individually, or may include at least two of the first to tenth switches simultaneously. That is, various combinations of switches are possible, with each switch individually controlling the components or circuits connected to its two ends. The specific number of switches and the switch combination method do not constitute a limitation on the scope of patent protection of the present disclosure.

In one embodiment, priority can be given to power supply from the power grid, and when the capacity of the power grid is insufficient, power can be supplied by both the power grid and the energy storage module or solely by the energy storage module, thereby reducing the usage frequency of the energy storage module and extending the lifespan of the supercapacitor 313.

In one embodiment, when the power distribution unit 21 includes the second inverter circuit 215, the other end of the supercapacitor 313 is further connected to the input terminal of the second inverter circuit 215; and the output terminal of the second inverter circuit 215 is connected to the stationary-side winding. In this embodiment, both inverter circuits are located on the stationary side, and the second inverter circuit 215 is powered by the supercapacitor 313.

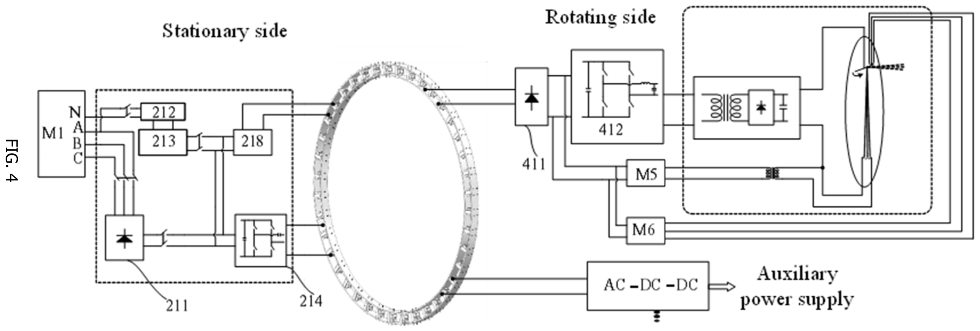

In one embodiment, referring to FIG. 4, wherein M1 represents a power grid, M5 represents a filament control unit, M6 represents an anode driver, the X-ray imaging apparatus further includes a third rectifier circuit 411, a third inverter circuit 412, and a rotating-side load. An input terminal of the third rectifier circuit 411 is connected to the rotating-side winding, an output terminal of the third rectifier circuit 411 is connected to an input terminal of the third inverter circuit 412, and an output terminal of the third inverter circuit 412 is connected to the rotating-side load. The power distribution unit 21 further includes a second DC/AC converter 218. The rotating-side load may include, but is not limited to, an HVG, a detector, etc.

An input terminal of the second DC/AC converter 218 is connected to the output terminal of the energy storage module 213 and/or the first rectifier circuit 211, and an output terminal of the second DC/AC converter 218 is connected to the stationary-side winding. When the power grid powers the wireless power transfer device solely, the second DC/AC converter 218 is used to convert a direct current voltage output by the first rectifier circuit 211 into an alternating current voltage and output the alternating current voltage to the stationary-side winding. The stationary-side winding then transfers the alternating current voltage to the rotating-side winding, and the rotating-side winding outputs the alternating current voltage to the rotating-side load via the third rectifier circuit 411 and the third inverter circuit 412. When the power grid and the energy storage module jointly power the wireless power transfer device, the second DC/AC converter 218 is used to convert a direct current voltage output by the first rectifier circuit 211 and/or the energy storage module into an alternating current voltage and output the alternating current voltage to the stationary-side winding. The stationary-side winding then transfers the alternating current voltage to the rotating-side winding, and the rotating-side winding outputs the alternating current voltage to the rotating-side load via the third rectifier circuit 411 and the third inverter circuit 412. When the energy storage module powers the wireless power transfer device solely, the second DC/AC converter 218 converts a direct current voltage output by the energy storage module into an alternating current voltage and outputs the alternating current voltage to the stationary-side winding. In this embodiment, the main power inversion is implemented on the rotating side, and the entire high-voltage system is arranged on the rotating side.

When the power grid is used to supply power solely, the three-phase power grid powers a second DC/AC circuit via the first rectifier circuit 211, and the second DC/AC circuit powers the stationary-side winding. Simultaneously, the first rectifier circuit 211 powers the first inverter circuit 214, and the first inverter circuit 214 powers the stationary-side winding. In one implementation, when the rotating-side winding includes a main winding and an auxiliary winding, the second DC/AC circuit and the first inverter circuit 214 respectively power the main winding and the auxiliary winding. The stationary-side winding transfers the alternating current voltage to the rotating-side winding, and the rotating-side winding supplies voltage to the HVG via the third rectifier circuit 411 and the third inverter circuit 412.

When the energy storage module 213 is used to supply power solely, the supercapacitor 313 powers the stationary-side winding via the second DC/AC circuit, and the supercapacitor 313 simultaneously powers the stationary-side winding via the first inverter circuit 214. In one implementation, when the rotating-side winding includes a main winding and an auxiliary winding, the second DC/AC circuit and the first inverter circuit 214 respectively power the main winding and the auxiliary winding. The stationary-side winding transfers the alternating current voltage to the rotating-side winding, and the rotating-side winding supplies voltage to the HVG via the third rectifier circuit 411 and the third inverter circuit 412.

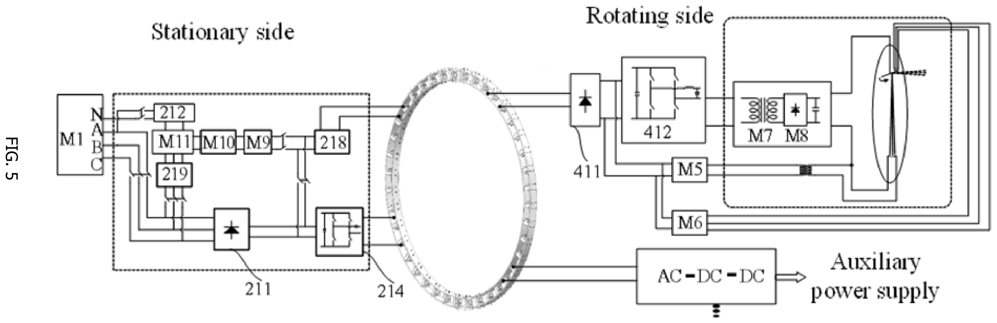

In one embodiment, referring to FIG. 5, wherein M1 represents a power grid, M5 represents a filament control unit, M6 represents an anode driver, M7 represents a rotating-side step-up transformer, M8 represents a rotating-side rectifier-filter module, M11 represents a rechargeable battery, M10 represents a bidirectional DC/DC converter, M9 represents a supercapacitor, the power distribution unit 21 further includes a third DC/AC converter 219. The rechargeable battery 311 is connected to the input terminal of the first rectifier circuit 211 via the third DC/AC converter 219. This embodiment is a further improvement on the power distribution unit 21 shown in FIG. 4. In the off-grid mode, the power grid stops powering the X-ray imaging apparatus, and the rechargeable battery 311 powers the first inverter circuit 214 via the third DC/AC converter 219 and the first rectifier circuit 211, and the first inverter circuit 214 powers the stationary-side winding. The rechargeable battery 311 can also power the second DC/AC converter 218 via the third DC/AC converter 219 and the first rectifier circuit 211.

In one implementation, when the rotating-side winding includes a main winding and an auxiliary winding, the second DC/AC circuit and the first inverter circuit 214 respectively power the main winding and the auxiliary winding. The stationary-side winding transfers the alternating current voltage to the rotating-side winding, and the rotating-side winding supplies voltage to the HVG via the third rectifier circuit 411 and the third inverter circuit 412.

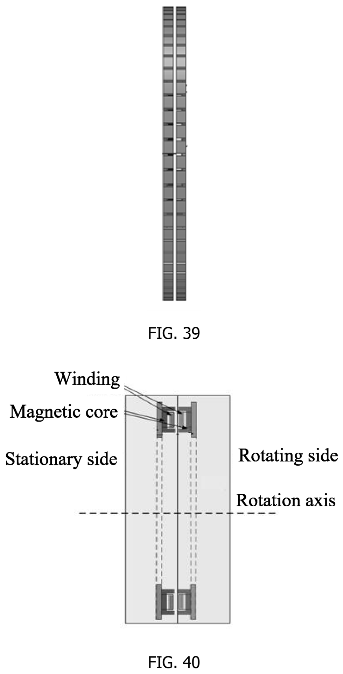

Those skilled in the art should understand that the power distribution unit of the present disclosure can also be used in conjunction with a wireless power transfer device of an existing non-contact slip ring structure. As shown in FIGS. 38-40, a magnetic core of the existing non-contact slip ring structure rotates together with the gantry, transferring electrical energy from the stationary side to the rotating side by means of electromagnetic coupling.

The inventors have found that since the wireless power transfer device transfers high-frequency electrical energy, electromagnetic interference problems arise. Electromagnetic interference would affect the communication interaction of the imaging apparatus, cause image distortion, and lead to certain harm to the human body due to excessive magnetic flux density at the rotation center. Moreover, when a hospital bed is made of metal, there may be eddy current effects that cause heat generation and other problems. In addition, for a wireless power transfer device employing a plurality of slip rings, magnetic fields of a plurality of coils couple with each other, affecting the transfer stability of high-frequency electrical energy.

In an optional implementation, the present disclosure further provides a wireless power transfer device, including: a first annular structure, on which a first frame is fixed, a first winding being wound around the first frame; and
a second annular structure, on which a second frame is fixed, a second winding being wound around the second frame.

The first winding and the second winding are disposed facing each other, and the second annular structure is rotatable relative to the first annular structure.

In an optional implementation, at least one of the first annular structure and the second annular structure is provided with a magnetic core assembly, through which wireless power transfer is achieved.

In an optional implementation, the magnetic core assembly is disposed on the first annular structure, and the first winding and the second winding share the magnetic core assembly. Therefore, the second annular structure does not need to be provided with a magnetic core assembly. In the solution of the present disclosure, fewer magnetic cores are provided, resulting in lower costs and faster heat dissipation, and heat generation of the winding can be mitigated during long-term high-power operation of the CT, thereby facilitating installation and maintenance and reducing costs. The magnetic core is not required to rotate with the second annular structure, reducing the weight of the gantry and allowing for higher rotational speeds.

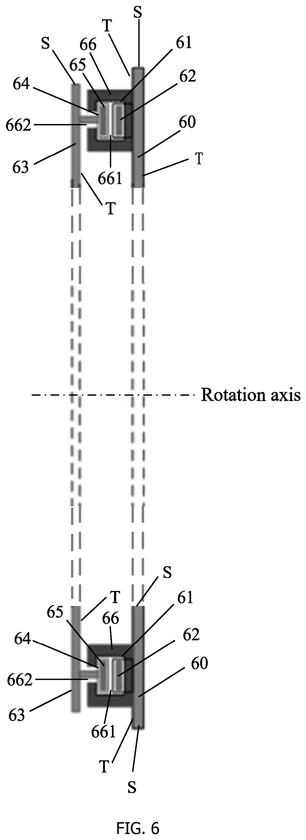

FIG. 6 is a structural schematic diagram of a wireless power transfer device according to an exemplary embodiment of the present disclosure. The wireless power transfer device includes: a first annular structure 60 and a second annular structure 63. A first frame 61 is fixed to an axial side surface T of the first annular structure 60, and a first winding 62 is wound around the first frame 61. A second frame 64 is fixed to an axial side surface of the second annular structure 63, the first frame 61 and the second frame 64 are disposed facing each other, and a second winding 65 is wound around the second frame 64. A gap exists between the first winding 62 and the second winding 65, and the second annular structure 63 can rotate relative to the first annular structure 60.

The axial direction of the annular structure is parallel to or coincides with a rotation axis of the wireless power transfer device. That is, the axial side surface T of the annular structure is a flat surface intersecting a circumferential surface S (including inner and outer circumferential surfaces) of the annular structure, or an extreme end surface of the circumferential surface S. The circumferential surface S is a closed curved surface of the annular structure.

In one embodiment, a magnetic core assembly is mounted to the axial side surface T of the first annular structure 60. The magnetic core assembly includes at least one magnetic core 66, and the first winding 62 passes through a window 661 of the magnetic core 66 of the magnetic core assembly. A second frame 64 is fixed to the axial side surface T of the second annular structure 63, and a second winding 65 is wound around the second frame 64. The second winding 65 passes through the window 661 of the magnetic core 66 of the magnetic core assembly. A gap exists between the first winding and the second winding disposed at the same magnetic core.

It should be noted that the number of first windings and the number of second windings included in the wireless power transfer device are not respectively limited to two as shown in FIG. 6, but can be set according to actual conditions. When there are a plurality of first windings and a plurality of second windings, the plurality of first windings may be dispersedly arranged on the axial side surface of the first annular structure. The plurality of first windings may be arranged in one circle, two circles, or even more. Correspondingly, the plurality of second windings are dispersedly arranged on the axial side surface of the second annular structure, and the plurality of second windings may be arranged in one circle, two circles, or even more. The arrangements of the first windings and the second windings are not specifically limited in the embodiments of the present disclosure.

In specific implementations, it is possible that a plurality of first frames 61 are fixed to the first annular structure 60, and a first winding 62 is wound around each first frame 61. It is also possible that one annular first frame 61 is fixed to the first annular structure 60, and a first winding 62 is wound around the first frame 61. The first winding 62 is an annular winding around the first annular structure 60.

Similarly, it is possible that a plurality of second frames 64 are fixed to the second annular structure 63, and a second winding 65 is wound around each second frame 64. It is also possible that one annular second frame 64 is fixed to the second annular structure 63, and a second winding 65 is wound around the second frame 64. The second winding 65 is an annular winding around the second annular structure 63.

The magnetic cores 66 of the magnetic core assembly may be non-uniformly mounted around the first annular structure 60. Preferably, the magnetic cores 66 of the magnetic core assembly are uniformly mounted to the circumference of the first annular structure 60. In this implementation, since the magnetic cores 66 of the magnetic core assembly are uniformly mounted around the first annular structure 60, the first frames 61, the first windings 62, and the second frames 64 corresponding to the magnetic cores 66 of the magnetic core assembly are also uniformly mounted accordingly, so that the wireless power transfer device can experience uniform stress, thereby improving the stability of the wireless power transfer device during operation.

In this embodiment, a certain gap is maintained between the first winding 62 and the second winding 65 to ensure that the first winding 62 and the second winding 65 do not contact each other when rotating relative to each other. The first winding 62 and the second winding 65 are magnetically coupled via the magnetic core assembly to transfer the electrical energy received by the first winding 62 to the second winding 65.

In one embodiment, when there are a plurality of first windings and a plurality of second windings, they can be classified as main windings and auxiliary windings. Specifically, part of the plurality of first windings serve as main windings, and the remaining first windings serve as auxiliary windings; and part of the plurality of second windings serve as main windings, and the remaining second windings serve as auxiliary windings.

For example, referring to FIG. 7, a first winding 62a and a second winding 65a serve as main windings, and a first winding 62b and a second winding 65b serve as auxiliary windings. In other implementations, it is also possible that the first winding 62a and the second winding 65a serve as auxiliary windings, and the first winding 62b and the second winding 65b serve as main windings.

The main windings and the auxiliary windings may be arranged regularly. Taking FIG. 7 as an example, the main windings are dispersedly arranged circumferentially in a first circle on an axial side surface of the annular structure, and the auxiliary windings are dispersedly arranged circumferentially in a second circle on the axial side surface of the annular structure. The diameter of the first circle is larger than the diameter of the second circle. This regular arrangement of the windings facilitates subsequent wiring to the power distribution unit. In other implementations, it is also possible that the windings are arranged irregularly. This is not specifically limited in the embodiments of the present disclosure.

In one embodiment, the second winding is connected to the inverter circuit of the power distribution unit, at which point the second winding serves as the stationary-side winding, and the first winding serves as the rotating-side winding.

In one embodiment, the first winding is connected to the inverter circuit of the power distribution unit, at which point the first winding serves as the stationary-side winding, and the second winding serves as the rotating-side winding. In this embodiment, the magnetic core is fixed to the first annular structure on the stationary side, rather than to the second annular structure on the rotating side. The magnetic core does not rotate with the second annular structure on the rotating side, thereby reducing the weight of the second annular structure on the rotating side and thus increasing the rotational speed of the second annular structure. Furthermore, since the magnetic core is fixed, the magnetic field can be kept stable, thereby improving the determinism of the wireless power transfer device.

In one embodiment, the X-ray imaging apparatus further includes: a gantry. The first annular structure 60 is fixedly connected to the gantry, and the second annular structure 63 can rotate relative to the first annular structure 60. In a specific embodiment, the first annular structure 60 is located on a stationary side of the gantry, and the second annular structure 63 is located on a rotating side of the gantry. During operation of the wireless power transfer device, the second annular structure 63 rotates with the gantry, and the first annular structure 60 and the second annular structure 63 rotate relative to each other. The stationary-side winding is connected to the power distribution unit. During the relative rotation of the first annular structure 60 and the second annular structure 63, the stationary-side winding transfers the alternating current voltage supplied by the power distribution unit to the rotating-side winding.

Furthermore, applying the wireless power transfer device according to this embodiment to the X-ray imaging apparatus can reduce the weight of the rotating side of the gantry, thereby facilitating an increase in the rotational speed of the gantry and improving temporal resolution of scans performed by the X-ray imaging apparatus.

In one embodiment, the magnetic core 66 of the magnetic core assembly has an air gap 662, and the second frame 64 passes through the air gap 662 and is fixed to the second annular structure 63. In this implementation, the stationary-side winding and the rotating-side winding can be magnetically coupled via the magnetic core assembly and the air gap 662 of the magnetic core 66 thereof, to transfer the electrical energy from the stationary-side winding to the rotating-side winding. The position of the air gap 662 in the magnetic core 66 determines the relative position between the second annular structure 63 and the first annular structure 60. Specifically, the structure of the magnetic core may be a UU-type, a UY-type, etc., and is not specifically limited herein.

The reduced-magnetic-core solution in the present disclosure can increase the cross-section of a single magnetic core while reducing the number of magnetic cores, thereby significantly reducing costs and making installation and maintenance more convenient. Furthermore, a larger space left between the magnetic cores allows for improved heat exchange efficiency for the windings, ensuring long-term high-power operation of the CT. However, the dispersed magnetic cores exhibit reduced capability to confine the magnetic flux lines, leading to an extended closure path for the magnetic flux lines. Moreover, in a dual-ring structure, inner and outer power rings exhibit mutual coupling, which would interfere with the operation of each other. The high-frequency, high magnetic induction intensity generated by the two power rings not only affects the information exchange of the CT but also exposes the patient to a high magnetic induction intensity. To address this issue, in an optional implementation, the present disclosure provides a wireless power transfer device with an electromagnetic shielding function.

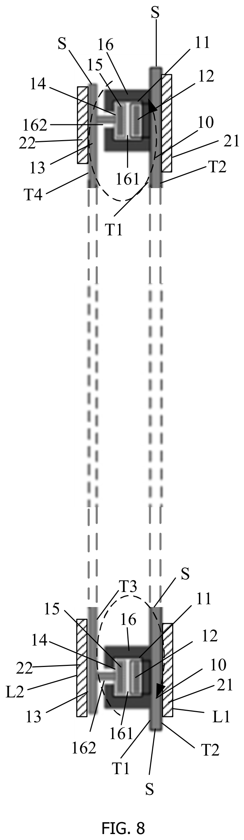

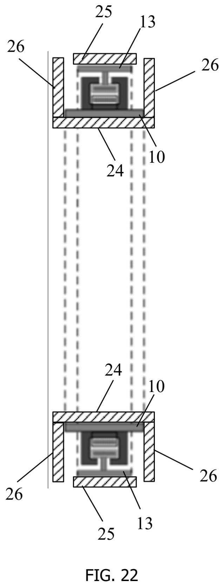

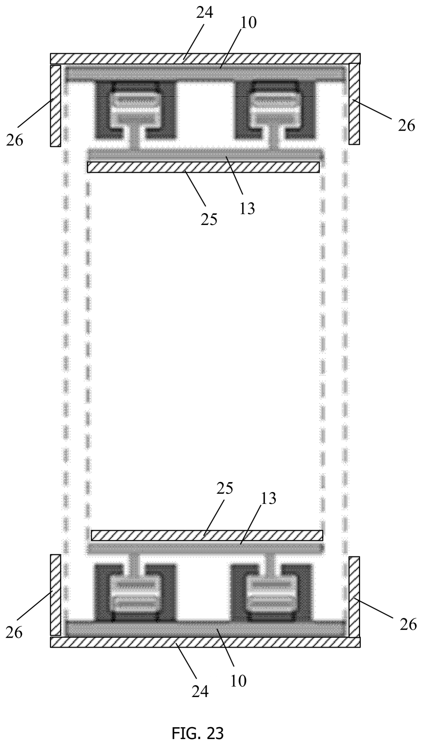

Referring to FIGS. 8-24, the wireless power transfer device includes: a first annular structure 10, a second annular structure 13, and a metal shielding assembly. A first frame 11 is fixed to the first annular structure 10, and a first winding 12 is wound around the first frame 11. A second frame 14 is fixed to the second annular structure 13, and a second winding 15 is wound around the second frame 14. The first winding 12 and the second winding 15 are disposed facing each other, and the second annular structure 13 can rotate relative to the first annular structure 10. The metal shielding assembly is disposed relative to the first winding 12 and/or the second winding 15. The metal shielding assembly can generate a reverse magnetic field based on the eddy current effect to weaken the main magnetic flux of the wireless power transfer device.

After an alternating current is passed through the windings, the magnetic field generated according to the law of electromagnetic induction can be constrained by a magnetic medium (such as the magnetic core), and form a closed loop along a designed main magnetic circuit, thereby realizing the magnetic flux of energy coupling/transfer. However, due to factors such as the limited permeability of the magnetic medium, the presence of an air gap in the magnetic circuit, and imperfect winding arrangements, part of the magnetic flux cannot be constrained by the main magnetic circuit and will "leak" from the main magnetic circuit (i.e., leakage flux). In this embodiment, the metal shielding assembly is disposed relative to the first winding and/or the second winding, and the leakage flux passing through the metal shielding assembly will induce eddy currents. According to Lenz's law, the eddy currents will generate a reverse magnetic field that is opposite to the direction of leakage flux. This reverse magnetic field can cancel out part of the leakage flux that spills outward, thereby reducing the leakage of magnetic field. This reduces electromagnetic interference and can also indirectly reduce magnetic flux loss to ensure the transfer efficiency of the main magnetic flux.

In one embodiment, the metal shielding assembly is disposed on an axial side surface of the first winding 12 and/or on an axial side surface of the second winding 15 to suppress magnetic leakage in horizontal and vertical directions of the windings.

In one embodiment, the metal shielding assembly is disposed between the first winding 12 and the second winding 15 to suppress magnetic leakage that permeates between the windings.

In one embodiment, the metal shielding assembly is disposed on the periphery of the first winding 12 and/or on the periphery of the first winding the second winding 15 to suppress magnetic leakage that diffuses to the periphery of the windings.

In this embodiment, adding the metal shielding assembly to the wireless power transfer device can utilize the eddy current effect of the metal material to reduce the magnetic field strength, thereby suppressing electromagnetic interference by changing the return path of the magnetic flux lines. This mitigates the impact of electromagnetic interference on the communication interaction of the X-ray imaging apparatus, reduces image distortion during scanning of the X-ray imaging apparatus, and also mitigates potential harm to the human body caused by excessive magnetic flux density at the rotation center of the wireless power transfer device and alleviates the heat generation and other problems caused by the eddy current effect.

In one embodiment, a magnetic core assembly is mounted to a first axial side surface T1 of the first annular structure 10. The magnetic core assembly includes at least one magnetic core 16, and the first winding 12 passes through a window 161 of the magnetic core 16 of the magnetic core assembly. A second frame 14 is fixed to a first axial side surface T3 of the second annular structure 13, and a second winding 15 is wound around the second frame 14. The second winding 15 passes through the window 161 of the magnetic core 16 of the magnetic core assembly. A gap exists between the first winding 12 and the second winding 15 disposed at the same magnetic core 16.

The first annular structure 10 includes two axial side surfaces, in which the first side surface T1 is a side surface close to the second annular structure 13, and a second side surface is a side surface away from the second annular structure 13. The second annular structure 13 includes two axial side surfaces, in which the first side surface T3 is a side surface close to the first annular structure 10, and a second side surface T4 is a side surface away from the first annular structure 10. That is, the axial side surface of the annular structure is a flat surface intersecting a circumferential surface S (including inner and outer circumferential surfaces) of the annular structure, or an extreme end surface of the circumferential surface S. The circumferential surface S is a closed curved surface of the annular structure.

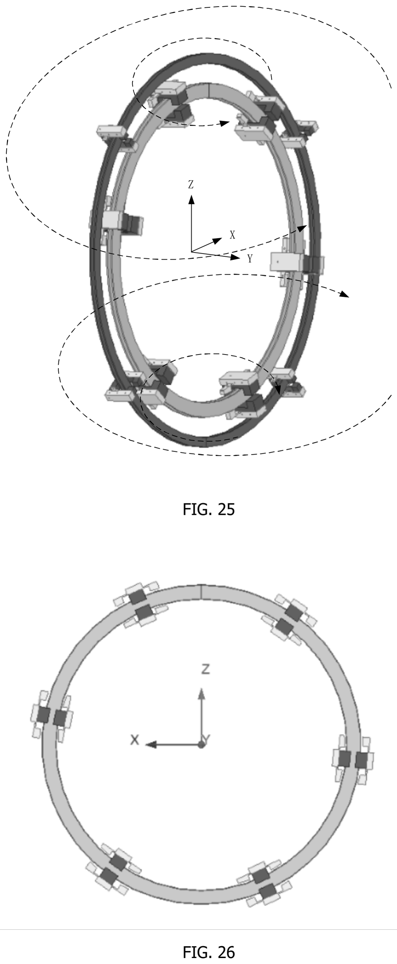

The axial directions of the first annular structure 10 and the second annular structure 13 are parallel to or coincide with the rotation axis of the wireless power transfer device. The axial directions of the first annular structure 10 and the second annular structure 13 are the Y-axis direction in FIGS. 25 and 26, the radial directions thereof are the X-axis direction or Z-axis direction in FIGS. 25 and 26, and the circumferential directions thereof are the rotation direction of the annular structure on the rotating side.

In one embodiment, the X-ray imaging apparatus further includes: a gantry. The first annular structure 10 is fixedly connected to the gantry, and the second annular structure 13 can rotate relative to the first annular structure 10. In a specific embodiment, the first annular structure 10 is located on a stationary side of the gantry, and the second annular structure 13 is located on a rotating side of the gantry. During operation of the wireless power transfer device, the second annular structure 13 rotates with the gantry, and the first annular structure 10 and the second annular structure 13 rotate relative to each other.

In this embodiment, the magnetic core is fixed to the first annular structure on the stationary side, rather than to the second annular structure on the rotating side. The magnetic core does not rotate with the second annular structure on the rotating side, thereby reducing the weight of the second annular structure on the rotating side and thus increasing the rotational speed of the second annular structure. Furthermore, since the magnetic core is fixed, the magnetic field can be kept stable, thereby ensuring stable power transfer.

In one embodiment, referring to FIG. 8, the magnetic core assembly is located at the first axial side surface T1 of the first annular structure 10. That is, the magnetic core 16 is located at the first axial side surface T1 of the first annular structure 10. To shield against electromagnetic interference, the metal shielding assembly includes a first metal shielding plate 21 located at the second axial side surface T2 of the first annular structure 10.

Referring to FIGS. 27 and 25, the magnetic flux lines (which are represented by the dashed lines with arrows in the figures) of the wireless power transfer device without the metal shielding assembly are widely distributed, and the number of magnetic flux lines is relatively large. Referring to FIG. 8, the provision of the first metal shielding plate 21 can suppress the outward expansion of the magnetic flux lines toward the first metal shielding plate 21, thereby achieving the purpose of shielding against electromagnetic interference. As the magnetic core of the first annular structure 10 on the stator side is closed, it provides the strongest capability to confine the magnetic flux lines. Adding the first metal shielding plate 21 to the second axial side surface T2 of the first annular structure 10 can significantly attenuate the strength of the peripheral magnetic field, thus achieving the purpose of effectively shielding against electromagnetic interference.

It should be noted that the magnetic core assembly may include a plurality of magnetic cores 16. The plurality of magnetic cores 16 may be irregularly mounted on the first axial side surface T1 of the first annular structure 10. Alternatively, the plurality of magnetic cores 16 may be uniformly mounted on the first axial side surface T1 of the first annular structure 10. When the plurality of magnetic cores 16 are uniformly mounted on the first axial side surface T1 of the first annular structure 10, the first frames 11, the first windings 12 and the second frames 14 corresponding to the magnetic cores 16 are also uniformly mounted accordingly, so that the wireless power transfer device can experience uniform stress, thereby improving the stability of the wireless power transfer device during operation.

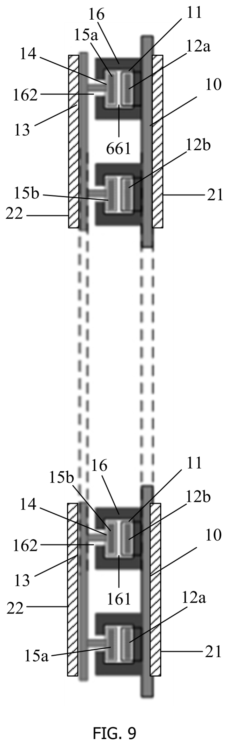

Referring to FIG. 8, a plurality of first windings 12 may be circumferentially mounted on the first axial side surface T1 of the annular structure in one circle to form one power ring. Alternatively, referring to FIGS. 9, 11, 13 and 15-20, a plurality of first windings 12 may be circumferentially mounted on the first axial side surface T1 of the annular structure in two circles of different diameters to form two power rings. Alternatively, a plurality of first windings 12 may be circumferentially mounted on the first axial side surface T1 of the annular structure in more circles of different diameters to form a plurality of power rings. The mounting method of the magnetic core 16 is not specifically limited in the embodiments of the present disclosure. It can be understood that the first frames 11, the second frames 14 and the magnetic cores 16 corresponding to the first windings 12 may also be circumferentially mounted in one circle, in two circles, or in a plurality of circles accordingly.

In one embodiment, when the magnetic core assembly includes a plurality of magnetic cores 16, there are a plurality of corresponding first windings and a plurality of corresponding second windings, which can be classified as main windings and auxiliary windings. Specifically, part of the plurality of first windings serve as main windings, and the remaining first windings serve as auxiliary windings; and part of the plurality of second windings serve as main windings, and the remaining second windings serve as auxiliary windings. For example, referring to FIGS. 9, 11, 13 and 15-20, a first winding 12a and a second winding 15a serve as main windings, and a first winding 12b and a second winding 15b serve as auxiliary windings. In other implementations, it is also possible that the first winding 12a and the second winding 15a serve as auxiliary windings, and the first winding 12b and the second winding 15b serve as main windings.

The size of the first metal shielding plate 21 is positively correlated with its electromagnetic interference suppression capability. In one embodiment, the area of an axial projection of the first metal shielding plate 21 is greater than or equal to the sum of the areas of projections of all the magnetic cores 16 on an axial side surface of the first metal shielding plate 21. Referring to FIG. 9, when the area of the axial projection of the first metal shielding plate 21 is greater than the sum of the areas of the projections of the magnetic core corresponding to the first winding 12a and the second winding 15a, and the magnetic core corresponding to the first winding 12b and the second winding 15b on the axial side surface of the first metal shielding plate 21, the effect of effectively suppressing electromagnetic interference can be achieved.

In one embodiment, the thickness of the first metal shielding plate 21 is negatively correlated with the number of magnetic cores. When the number of magnetic cores is small, the electromagnetic interference suppression capability of the first metal shielding plate 21 can be enhanced by increasing the thickness of the first metal shielding plate 21.

To mitigate the heat generation of the winding during long-term high-power operation of the CT, facilitate installation and maintenance, and reduce costs, the number of magnetic cores can be reduced. The reduced-magnetic-core solution significantly reduces costs and makes installation and maintenance more convenient. Furthermore, a larger space can be left between the magnetic cores to allow for improved heat exchange efficiency for the windings, ensuring long-term high-power operation of the X-ray imaging apparatus. However, reducing the number of magnetic cores implies a dispersed arrangement of the magnetic cores. Such dispersed magnetic cores exhibit a reduced capability to confine the magnetic flux lines, causing the closure path of the magnetic flux lines to become longer.

In this embodiment, the thickness of the first metal shielding plate 21 is determined based on the number of magnetic cores. This can enhance the electromagnetic interference suppression capability and also effectively address the heat generation problem of the winding.

In one embodiment, the metal shielding assembly includes a second metal shielding plate 22. The second frame 14 is fixed to the first axial side surface T3 of the second annular structure 13, and the second metal shielding plate 22 is disposed on the second axial side surface T4 of the second annular structure 13.

In one embodiment, a connection part between the gantry and a fixing structural member of the second annular structure 13 is designed as an annular structure to act as the second metal shielding plate 22. In other implementations, if structural constraints exist, for example, the gantry has a limited coverage area or is far away from the magnetic core, a separate metal shielding plate may be added.

Similar to the first metal shielding plate 21, the area of an axial projection of the second metal shielding plate 22 is greater than or equal to the sum of the areas of projections of all the magnetic cores 16 on an axial side surface of the second metal shielding plate 22.

In one embodiment, the thickness of the second metal shielding plate 22 is negatively correlated with the number of magnetic cores. When the number of magnetic cores is small, the electromagnetic interference suppression capability of the second metal shielding plate 22 can be enhanced by increasing the thickness of the second metal shielding plate 22.

In one embodiment, referring to FIG. 8, simultaneously providing the first metal shielding plate 21 in the first annular structure 10 and the second metal shielding plate 22 in the second annular structure 13 can effectively weaken the strength of the external magnetic field.

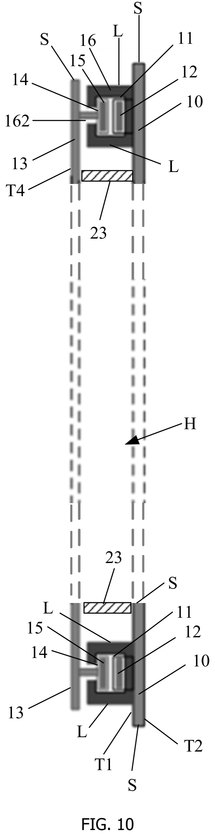

In one embodiment, referring to FIGS. 10-20, the metal shielding assembly includes a first metal shielding ring structure 23. When the wireless power transfer device includes a magnetic core 16, the first metal shielding ring structure 23 is fitted over at least one circumferential surface L of the magnetic core 16. When the wireless power transfer device includes no magnetic core 16, the first metal shielding ring structure 23 is fitted over at least one circumferential surface of a power ring, which is formed by a plurality of first windings 12 circumferentially arranged on the first axial side surface T1 of the first annular structure 10.

In one embodiment, referring to FIG. 10, a plurality of magnetic cores 16 are circumferentially mounted on the first axial side surface T1 of the first annular structure in one circle to form one power ring, and the first metal shielding ring structure 23 is fitted over an inner circumferential surface of the power ring. That is, the first metal shielding ring structure 23 is located within a through hole H formed by the inner circumferential surface of the power ring. The diameter of the inner circumferential surface of the power ring is smaller than the diameter of an outer circumferential surface of the power ring.

In other implementations, the first metal shielding ring structure 23 is fitted over the outer circumferential surface of the power ring, or a first metal shielding ring structure 23 is fitted over each of the inner and outer circumferential surfaces of the magnetic core 16.

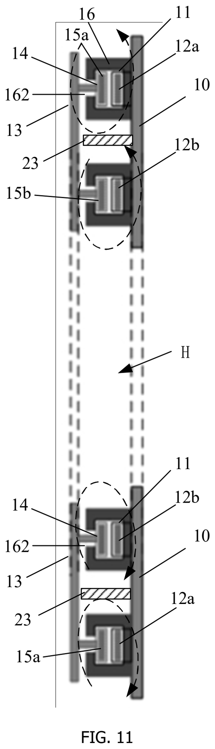

In one embodiment, a plurality of magnetic cores 16 are circumferentially mounted on the first axial side surface T1 of the first annular structure in at least two circles of different diameters, with the magnetic cores 16 in each circle forming one power ring. Taking two power rings (a plurality of magnetic cores 16 circumferentially mounted on the first axial side surface T1 of the first annular structure in two circles of different diameters) shown in FIG. 11 as an example, the magnetic cores corresponding to all first windings 12a and second windings 15a form one power ring, and the magnetic cores corresponding to all first windings 12b and second windings 15b form one power ring. The first metal shielding ring structure 23 is disposed between the magnetic core corresponding to the first winding 12a and the second winding 15a and the magnetic core corresponding to the first winding 12b and the second winding 15b. That is, the first metal shielding ring structure 23 is fitted over the inner circumference of the magnetic core corresponding to the first winding 12a and the second winding 15a, or the first metal shielding ring structure 23 is fitted over the outer circumference of the magnetic core corresponding to the first winding 12b and the second winding 15b.

For a wireless power transfer device including a plurality of power rings, due to device size limitations, the power rings are too close to each other, resulting in coupling problems. In this embodiment, the provision of the first metal shielding ring structure between two adjacent power rings can not only suppress electromagnetic interference but can also reduce coupling between two adjacent power rings.

Alternatively, in other implementations, the first metal shielding ring structure 23 may be fitted over the outer circumference of the magnetic core corresponding to the first winding 12a and the second winding 15a, and/or the inner circumference of the magnetic core corresponding to the first winding 12b and the second winding 15b.

In one embodiment, the thickness of the first metal shielding ring structure 23 is negatively correlated with the number of magnetic cores. When the number of magnetic cores is small, the electromagnetic interference suppression capability of the first metal shielding ring structure 23 can be enhanced by increasing the thickness of the first metal shielding ring structure 23.

To mitigate the heat generation of the winding during long-term high-power operation of the CT, facilitate installation and maintenance, and reduce costs, the number of magnetic cores can be reduced. The reduced-magnetic-core solution significantly reduces costs and makes installation and maintenance more convenient. Furthermore, a larger space can be left between the magnetic cores to allow for improved heat exchange efficiency for the windings, ensuring long-term high-power operation of the X-ray imaging apparatus. However, reducing the number of magnetic cores implies a dispersed arrangement of the magnetic cores. Such dispersed magnetic cores exhibit a reduced capability to confine the magnetic flux lines, causing the closure path of the magnetic flux lines to become longer. Furthermore, in a multi-power-ring structure, adjacent power rings exhibit mutual coupling, which would interfere with the operation of each other.

In view of this, in this embodiment, the thickness of the first metal shielding ring structure 23 is increased to enhance the electromagnetic interference suppression capability of the first metal shielding ring structure 23 and further reduce coupling between two adjacent power rings.

The first metal shielding ring structure 23 may be fixed to the first axial side surface T1 of the first annular structure 10, or the first metal shielding ring structure 23 may be fixed to the gantry.

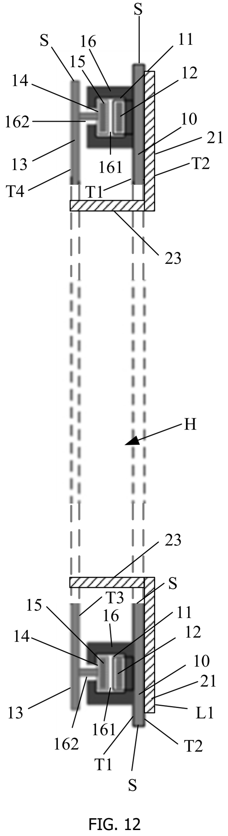

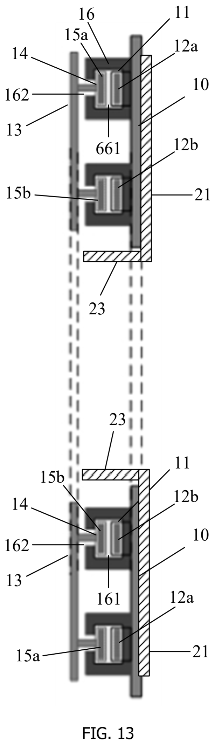

Referring to FIGS. 12 and 13, when the metal shielding assembly includes both a first metal shielding plate 21 and a first metal shielding ring structure 23, the first metal shielding ring structure 23 may be fixed to the first metal shielding plate 21, at which point the cross-section of the metal shielding assembly is L-shaped. Preferably, an axial side surface of the first metal shielding ring structure close to the first metal shielding plate is seamlessly connected to the first metal shielding plate, thereby ensuring the integrity of the metal shielding assembly, preventing electromagnetic leakage through a slit in the metal shielding assembly, and further improving the electromagnetic interference suppression capability of the metal shielding assembly.

When the metal shielding assembly includes both a second metal shielding plate 22 and a first metal shielding ring structure 23, the first metal shielding ring structure 23 may be fixed to the second metal shielding plate 22, at which point the cross-section of the metal shielding assembly is L-shaped. Preferably, an axial side surface of the first metal shielding ring structure close to the second metal shielding plate is seamlessly connected to the second metal shielding plate, thereby further improving the electromagnetic interference suppression capability of the metal shielding assembly.

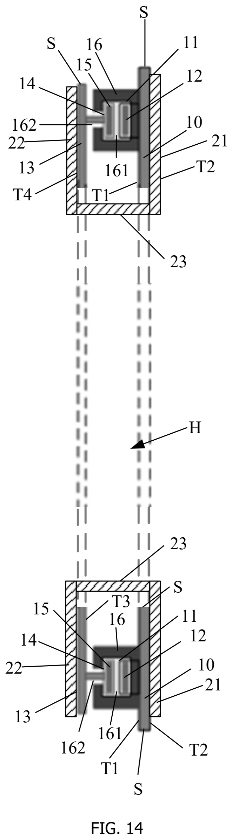

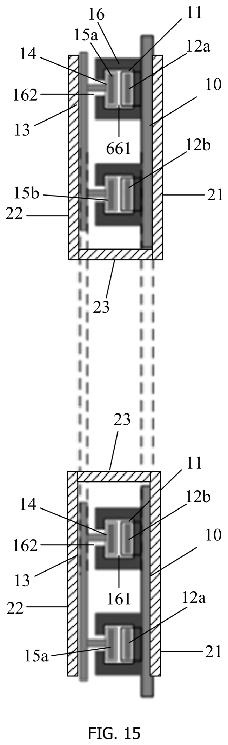

Referring to FIGS. 14 and 15, when the metal shielding assembly includes a first metal shielding plate 21, a second metal shielding plate 22, and a first metal shielding ring structure 23, the two axial side surfaces of the first metal shielding ring structure 23 may be respectively fixed to the first metal shielding plate 21 and the second metal shielding plate 22, at which point the cross-section of the metal shielding assembly is U-shaped.

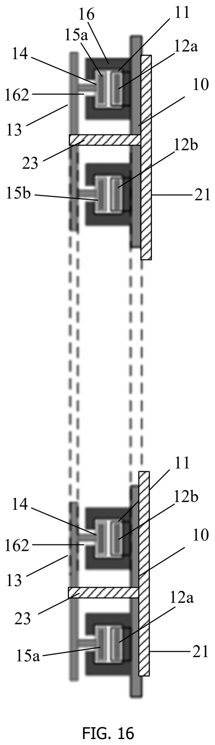

Referring to FIG. 16, when the metal shielding assembly includes both a first metal shielding plate 21 and a first metal shielding ring structure 23, and the first metal shielding ring structure 23 is located between two power rings (a power ring formed by the magnetic core corresponding to the first winding 12a and the second winding 15a, and a power ring formed by the magnetic core corresponding to the first winding 12b and the second winding 15b), the end of the first metal shielding ring structure 23 close to the first annular structure 10 may pass through a through hole in the first annular structure 10 and be fixed to the first metal shielding plate 21, at which point the cross-section of the metal shielding assembly is T-shaped.

In other implementations, when the metal shielding assembly includes both a second metal shielding plate 22 and a first metal shielding ring structure 23, and the first metal shielding ring structure 23 is located between two power rings, the end of the first metal shielding ring structure 23 close to the second annular structure 13 may pass through a through hole in the second annular structure 13 and be fixed to the second metal shielding plate 22, at which point the cross-section of the metal shielding assembly is T-shaped. The specific implementation is similar to that in FIG. 16 and will not be described again here.

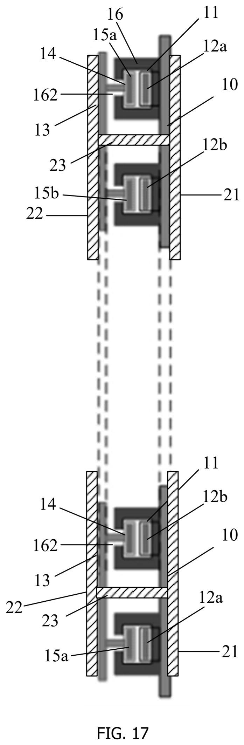

Referring to FIG. 17, the wireless power transfer device includes two power rings. When the metal shielding assembly includes a first metal shielding plate 21, a second metal shielding plate 22, and a first metal shielding ring structure 23, and the first metal shielding ring structure 23 is located between the two power rings, the first metal shielding ring structure 23 may be fixed to both the first metal shielding plate 21 and the second metal shielding plate 22, at which point the cross-section of the metal shielding assembly is H-shaped. The metal shielding assembly in this shape not only increases the closure path of the coupled magnetic flux lines of each power ring, but also significantly attenuates the strength of the magnetic field after the peripheral magnetic flux lines pass through the metal shielding plate, thereby reducing the coupling between the two power rings. This embodiment simultaneously solves the problems of excessive central magnetic flux density and coupling between adjacent power rings.

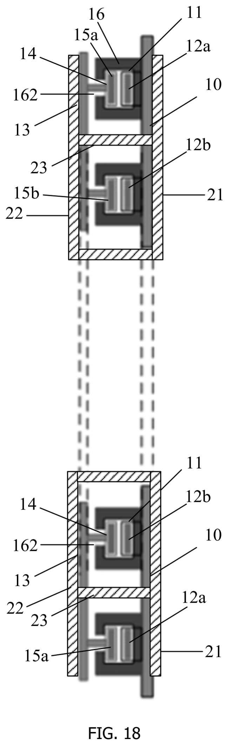

Referring to FIG. 18, the wireless power transfer device includes two power rings. When the metal shielding assembly includes a first metal shielding plate 21, a second metal shielding plate 22, and two metal shielding ring structures 23, and the two metal shielding ring structures 23 are respectively fitted over an inner circumferential surface of the smaller-diameter power ring and between the two power rings, the two metal shielding ring structures 23 may be fixed to both the first metal shielding plate 21 and the second metal shielding plate 22.

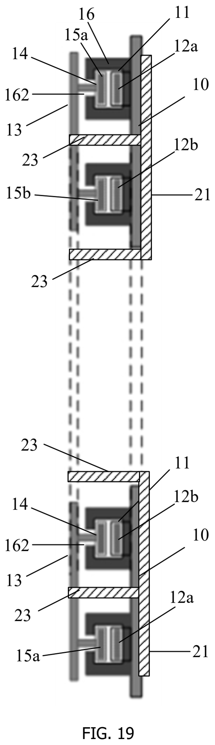

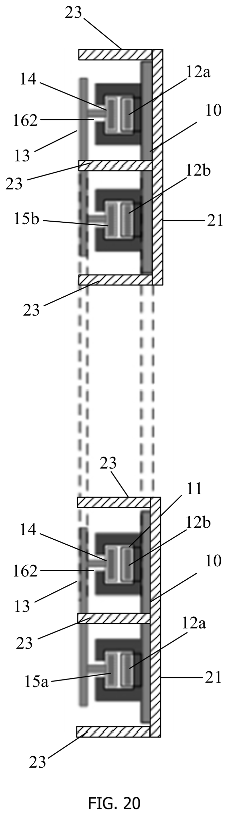

It should be noted that the first metal shielding plate 21, the second metal shielding plate 22, and the first metal shielding ring structure 23 can be combined according to actual requirements to achieve the desired electromagnetic interference shielding. Alternatively, in other implementations, referring to FIGS. 19 and 20, the cross-section of the metal shielding assembly may be F-shaped or E-shaped. The E-shaped metal shielding assembly forms a semi-enclosed metal shielding plate, which can significantly reduce the central magnetic flux density.

It can be understood that since the magnetic core has high permeability on its closed surface and the strongest ability to confine the magnetic flux lines, adding a metal shielding plate and/or a metal shielding ring structure to the closed surface of the magnetic core can effectively suppress electromagnetic interference.

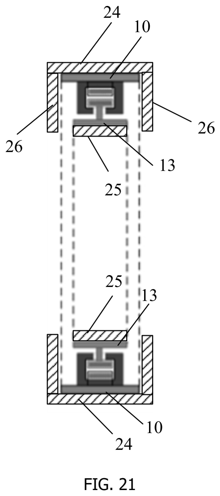

In one embodiment, referring to FIGS. 21-23, the magnetic core is located on a first circumferential surface of the first annular structure 10, that is, the first frame 11, around which the first winding 12 is wound, is located on the first circumferential surface of the first annular structure 10. The metal shielding assembly includes a second metal shielding ring structure 24. The second metal shielding ring structure 24 is fitted over a second circumferential surface of the first annular structure 10. The first circumferential surface of the first annular structure 10 may be its inner circumferential surface, and correspondingly, the second circumferential surface of the first annular structure 10 is its outer circumferential surface. Alternatively, the first circumferential surface of the first annular structure 10 may be its outer circumferential surface, and correspondingly, the second circumferential surface of the first annular structure 10 is its inner circumferential surface.

In one embodiment, the second frame 14, around which the second winding 15 is wound, is located on a first circumferential surface of the second annular structure 13, and the second winding 15 and the first winding 12 are disposed facing each other. That is, when the first winding 12 is located on the inner circumferential surface of the first annular structure 10, the second winding 15 is located on the outer circumferential surface of the second annular structure 13, at which point the outer circumferential surface of the second annular structure 13 is its first circumferential surface and the inner circumferential surface of the second annular structure 13 is its second circumferential surface, and a second metal shielding ring structure may be fitted over the second circumferential surface (the inner circumferential surface) of the second annular structure 13. When the first winding 12 is located on the outer circumferential surface of the first annular structure 10, the second winding 15 is located on the inner circumferential surface of the second annular structure 13, at which point the inner circumferential surface of the second annular structure 13 is its first circumferential surface and the outer circumferential surface of the second annular structure 13 is its second circumferential surface, and a second metal shielding ring structure may be fitted over the second circumferential surface (the outer circumferential surface) of the second annular structure 13.

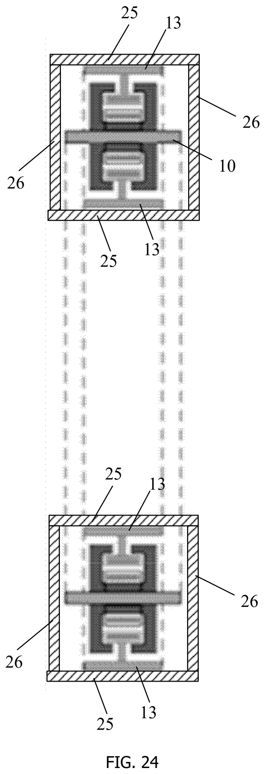

In one embodiment, referring to FIG. 24, both circumferential surfaces of the first annular structure 10 are provided with magnetic cores, that is, both the inner and outer circumferential surfaces of the first annular structure 10 are provided with magnetic cores and first frames. Correspondingly, two second annular structures 13 are provided, and a second frame is fixed to the first circumferential surface of each second annular structure 13 relative to the first frame. The metal shielding assembly includes a third metal shielding ring structure 25. One third metal shielding ring structure 25 is fitted over the second circumferential surface of at least one of the second annular structures 13. In FIG. 24, one third metal shielding ring structure 25 is fitted over the second circumferential surface of each of the two second annular structures 13. Alternatively, in other implementations, one third metal shielding ring structure 25 may be fitted only over the outer circumferential surface of the second annular structure 13 with a larger diameter, or one third metal shielding ring structure 25 may be fitted only over the inner circumferential surface of the second annular structure 13 with a smaller diameter.

In one embodiment, referring to FIGS. 21-24, the metal shielding assembly includes a third metal shielding plate 26. The third metal shielding plate 26 is disposed opposite a first and/or second axial side surface of the first annular structure 10.

It should be noted that in practical applications, the second metal shielding ring structure 24, the third metal shielding ring structure 25, and the third metal shielding plate 26 are not limited to the combination shown in FIGS. 21-24. The second metal shielding ring structure 24, the third metal shielding ring structure 25, and the third metal shielding plate 26 may be used independently or in pairs.

In one embodiment, an axial side surface of the second metal shielding ring structure 24 is seamlessly connected to the third metal shielding plate 26.

In one embodiment, an axial side surface of the third metal shielding ring structure 25 is seamlessly connected to the third metal shielding plate 26.

In one embodiment, the area of an axial projection of the third metal shielding plate 26 is greater than or equal to the area of a projection of the magnetic core on an axial side surface of the third metal shielding plate 26.

In one embodiment, the area of a radial projection of the second metal shielding ring structure 24 is greater than or equal to the area of a projection of the magnetic core on a circumferential surface of the second metal shielding ring structure 24.

In one embodiment, the area of a radial projection of the third metal shielding ring structure 25 is greater than or equal to the area of a projection of the magnetic core on a circumferential surface of the third metal shielding ring structure 25.

The specific implementations of the second metal shielding ring structure 24, the third metal shielding ring structure 25, and the third metal shielding plate 26 are similar to those of the first metal shielding plate 21, the second metal shielding ring structure 22, and the first metal shielding ring structure 23, and will not be described again here.

In one embodiment, the metal shielding assembly is made of aluminum. Aluminum has good conductivity and high electromagnetic shielding effectiveness. Furthermore, aluminum is lightweight, and will not significantly affect the weight of the second annular structure (the rotating side) even if a metal shielding plate is added to the second annular structure (the rotating side), without affecting the rotational speed of the second annular structure (the rotating side).

In one embodiment, a certain gap is maintained between the first winding 12 and the second winding 15 to ensure that the first winding 12 and the second winding 15 do not contact each other when rotating relative to each other. The first winding 12 and the second winding 15 are magnetically coupled via the magnetic core assembly to transfer the electrical energy received by the first winding 12 to the second winding 15.

In one embodiment, the magnetic core 16 of the magnetic core assembly has an air gap 162, and the second frame 14 passes through the air gap 162 and is fixed to the second annular structure 13. In this implementation, the stationary-side winding and the rotating-side winding can be magnetically coupled via the magnetic core assembly and the air gap 162 of the magnetic core 16 thereof, to transfer the electrical energy received by the stationary-side winding to the rotating-side winding. The position of the air gap 162 in the magnetic core 16 determines the relative position between the second annular structure 13 and the first annular structure 10. Specifically, the structure of the magnetic core may be a UU-type, a UY-type, etc., and is not specifically limited herein.

Those skilled in the art should understand that the wireless power transfer device in the above embodiments of the present disclosure can exist independently of the power distribution unit in the above embodiments of the present disclosure, or it can be used in conjunction with the power distribution unit in the above embodiments of the present disclosure.

The embodiments of the present disclosure further provide an X-ray imaging apparatus, which includes the wireless power transfer device according to any of the above embodiments and/or the power distribution unit according to any of the above embodiments.

In an optional implementation, the power distribution unit is connected to the wireless power transfer device; and the power distribution unit is used to power the wireless power transfer device. The specific implementation of the wireless power transfer device and the specific power supply method of the power distribution unit refer to the description of any of the above embodiments, and will not be described again here.

In an optional implementation, the X-ray imaging apparatus includes the wireless power transfer device according to any of the above embodiments and a gantry. The first annular structure is fixedly connected to the gantry and is located on a stationary side of the gantry, and the second annular structure is located on a rotating side of the gantry.

In an optional implementation, when the X-ray imaging apparatus further includes the power distribution unit, the power distribution unit is connected to the first winding. In this case, the first annular structure corresponding to the first winding serves as the stationary side, and the second annular structure corresponding to the second winding serves as the rotating side.

The X-ray imaging apparatus can be a CT device, or a combined CT and other modal imaging apparatus, such as a PET-CT device combining CT and PET (positron emission tomography).

Although the specific implementations of the present disclosure are described above, it should be appreciated by those skilled in the art that these are merely illustrative and that the scope of protection of the present disclosure is defined by the appended claims. Various changes or modifications to these implementations may be made by those skilled in the art without departing from the principle and spirit of the present disclosure, and these changes or modifications fall within the scope of protection of the present disclosure.

## Claims

1. A wireless power transfer device, comprising:
a first annular structure (60, 10), on which a first frame (61, 11) is fixed, a first winding (62, 12) being wound around the first frame (61, 11); and
a second annular structure (63, 13), on which a second frame (64, 14) is fixed, a second winding (65, 15) being wound around the second frame (64, 14),
wherein the first winding (62, 12) and the second winding (65, 15) are disposed facing each other, and the second annular structure (63, 13) is rotatable relative to the first annular structure (60, 10).

2. The wireless power transfer device according to claim 1, wherein the first frame (61) is fixed to an axial side surface (T) of the first annular structure (60), and the second frame (64) is fixed to an axial side surface (T) of the second annular structure (63); and
a gap exists between the first winding (62) and the second winding (65).

3. The wireless power transfer device according to claim 2, wherein the wireless power transfer device comprises at least two first windings (62), part of the at least two first windings (62) being main windings and the remaining first windings (62) being auxiliary windings; the wireless power transfer device comprises at least two second windings (65), part of the at least two second windings (65) being main windings and the remaining second windings (65) being auxiliary windings;
and/or, a magnetic core assembly is further mounted to an axial side surface (T) of the first annular structure (60), the magnetic core assembly comprising at least one magnetic core (66), the first winding (62) passing through a window (661) of the magnetic core (66), and the second frame (64) passing through an air gap (662) of the magnetic core (66) and being fixed to the second annular structure (63);
and/or, the first annular structure (60) and the second annular structure (63) have the same dimensions.

4. The wireless power transfer device according to claim 1, wherein the wireless power transfer device has an electromagnetic shielding function; the wireless power transfer device further comprising:
a metal shielding assembly disposed relative to the first winding (12) and/or the second winding (15);
and/or, the metal shielding assembly is disposed on an axial side surface of the first winding (12) and/or on an axial side surface the second winding (15),
and/or, the metal shielding assembly is disposed between the first winding (12) and the second winding (15),
and/or, the metal shielding assembly is disposed on the periphery of the first winding (12) and/or on the periphery of the second winding (15).

5. The wireless power transfer device according to claim 4, wherein the first frame (11), around which the first winding (12) is wound, is fixed to a first axial side surface (T1) of the first annular structure (10); and the metal shielding assembly comprises at least one of a first metal shielding plate (21), a second metal shielding plate (22), and a first metal shielding ring structure (23);
wherein the first metal shielding plate (21) disposed on a second axial side surface (T2) of the first annular structure (10);
and/or, the second frame (14), around which the second winding (15) is wound, is fixed to a first axial side surface (T3) of the second annular structure (13), and the second metal shielding plate (22) is disposed on a second axial side surface (T4) of the second annular structure (13);
and/or, the wireless power transfer device comprises a plurality of first windings (12), which are circumferentially arranged on the first axial side surface (T1) of the first annular structure (10) to form a power ring, the first metal shielding ring structure (23) being fitted over at least one circumferential surface of the power ring, wherein an axis of the power ring coincides with an axis of the first annular structure (10).

6. The wireless power transfer device according to claim 5, wherein optionally, when the metal shielding assembly comprises the first metal shielding ring structure (23) and the first metal shielding plate (21), an axial side surface of the first metal shielding ring structure (23) close to the first metal shielding plate (21) is seamlessly connected to the first metal shielding plate (21);
wherein optionally, when the metal shielding assembly comprises the first metal shielding ring structure (23) and the second metal shielding plate (22), an axial side surface of the first metal shielding ring structure (23) close to the second metal shielding plate (22) is seamlessly connected to the second metal shielding plate (22).

7. The wireless power transfer device according to claim 5 or claim 6, wherein a magnetic core assembly is further mounted to the first axial side surface (T1) of the first annular structure (10), the magnetic core assembly comprising at least one magnetic core (16), the first winding (12) passing through a window (161) of the magnetic core (16), and the second frame (14) passing through an air gap (162) of the magnetic core (16) and being fixed to the second annular structure (13);
wherein the area of an axial projection of the first metal shielding plate (21) is greater than or equal to the area of a projection of the magnetic core (16) on an axial side surface of the first metal shielding plate (21);
and/or, the area of an axial projection of the second metal shielding plate (22) is greater than or equal to the area of a projection of the magnetic core (16) on an axial side surface of the second metal shielding plate (22);
and/or, the area of a radial projection of the first metal shielding ring structure (23) is greater than or equal to the area of a projection of the magnetic core (16) on a circumferential surface of the first metal shielding ring structure (23).

8. The wireless power transfer device according to claim 4, wherein the first frame (11), around which the first winding (12) is wound, is located on a first circumferential surface of the first annular structure (10); the metal shielding assembly comprises a second metal shielding ring structure (24); the second metal shielding ring structure (24) is fitted over a second circumferential surface of the first annular structure (10) and/or a first circumferential surface of the second annular structure (13); the second frame (14) is fixed to a second circumferential surface of the second annular structure (13);
and/or, each circumferential surface of the first annular structure (10) is provided with a first frame (11), and one second annular structure (13) is provided opposite each circumferential surface of the first annular structure (10), a second frame (14) being fixed to the first circumferential surface of each second annular structure (13) relative to the first frame (11); the metal shielding assembly comprises a third metal shielding ring structure (25); one third metal shielding ring structure (25) is fitted over the second circumferential surface of at least one of the second annular structures (13);
and/or, the metal shielding assembly comprises a third metal shielding plate (26); and the third metal shielding plate (26) is disposed opposite a first and/or second axial side surface of the first annular structure (10).

9. The wireless power transfer device according to claim 8, wherein optionally, when the metal shielding assembly comprises the second metal shielding ring structure (24) and the third metal shielding plate (26), an axial side surface of the second metal shielding ring structure (24) is seamlessly connected to the third metal shielding plate (26);
wherein optionally, when the metal shielding assembly comprises the third metal shielding ring structure (25) and the third metal shielding plate (26), an axial side surface of the third metal shielding ring structure (25) is seamlessly connected to the third metal shielding plate (26).

10. The wireless power transfer device according to claim 8 or claim 9, wherein a magnetic core assembly is further mounted to the first axial side surface (T1) of the first annular structure (10), the magnetic core assembly comprising at least one magnetic core (16), the first winding (12) passing through a window (161) of the magnetic core (16), and the second frame (14) passing through an air gap (162) of the magnetic core (16) and being fixed to the second annular structure (13);
wherein the area of an axial projection of the third metal shielding plate (26) is greater than or equal to the area of a projection of the magnetic core (16) on an axial side surface of the third metal shielding plate (26);
and/or, the area of a radial projection of the second metal shielding ring structure (24) is greater than or equal to the area of a projection of the magnetic core (16) on a circumferential surface of the second metal shielding ring structure (24);
and/or, the area of a radial projection of the third metal shielding ring structure (25) is greater than or equal to the area of a projection of the magnetic core (16) on a circumferential surface of the third metal shielding ring structure (25).

11. The wireless power transfer device according to any one of preceding claims 4-10, wherein the metal shielding assembly is made of aluminum.

12. The wireless power transfer device according to any one of preceding claims 4-11, wherein a magnetic core assembly is further mounted to a first axial side surface (T1) of the first annular structure (10), the magnetic core assembly comprising at least one magnetic core (16), the first winding (12) passing through a window (161) of the magnetic core (16), and the second frame (14) passing through an air gap (162) of the magnetic core (16) and being fixed to the second annular structure (13); and the thickness of the metal shielding assembly is negatively correlated with the number of magnetic cores (16).

13. The wireless power transfer device according to any one of preceding claims 4-12, wherein the first annular structure (10) is configured for fixed connection with a gantry, and is located on a stationary side of the gantry.

14. An X-ray imaging apparatus, comprising: the wireless power transfer device according to any one of preceding claims 1-13;
wherein optionally, the X-ray imaging apparatus further includes: a gantry; wherein the first annular structure (60, 10) is fixedly connected to the gantry, the first annular structure (60, 10) is located on a stationary side of the gantry, and the second annular structure (63, 13) is located on a rotating side of the gantry.

15. The X-ray imaging apparatus according to claim 14, wherein the X-ray imaging apparatus further includes a power distribution unit (21);
the power distribution unit (21) comprising:
a first rectifier circuit (211), a charging circuit (212), an energy storage module (213), and a first inverter circuit (214);
wherein input terminals of the first rectifier circuit (211) and input terminals of the charging circuit (212) are both externally connected to a power grid;
wherein an output terminal of the first rectifier circuit (211) is connected to an input terminal of the first inverter circuit (214), and an output terminal of the first inverter circuit (214) is connected to the wireless power transfer device;
wherein an output terminal of the charging circuit (212) is connected to one end of the energy storage module (213), and the other end of the energy storage module (213) is connected to the input terminal of the first inverter circuit (214);
wherein the first inverter circuit (214) is configured to convert an output voltage of the first rectifier circuit (211) into an alternating current voltage and output the alternating current voltage to the wireless power transfer device;
wherein optionally, the power distribution unit (21) is connected to the first winding (62, 12).
